# EUROPÄISCHE PATENTSCHRIFT

(11) **EP 3 965 689 B1**
(45) Veröffentlichungstag und Bekanntmachung des Hinweises auf die Patenterteilung: **21.08.2024**
(21) Anmeldenummer: 20724083.9
(22) Anmeldetag: 05.05.2020
(51) Int. Cl.: A61F 2/06, A61F 2/07, A61F 2/24

(54) **GEFÄSSIMPLANTAT UND VERFAHREN ZU DESSEN HERSTELLUNG**
BLOOD VESSEL IMPLANT AND METHOD FOR THE PRODUCTION THEREOF
IMPLANT VASCULAIRE ET PROCÉDÉ POUR SA FABRICATION

(30) Priorität: 06.05.2019 DE 102019206493
(43) Veröffentlichungstag der Anmeldung: 16.03.2022
(73) Patentinhaber: Rheinisch-Westfälische Technische Hochschule (RWTH) Aachen, 52062 Aachen (DE)
(72) Erfinder: GESCHÉ, Valentine, 81369 München (DE); LÖWEN, Alexander, 52066 Aachen (DE); KURTENBACH, Kathrin, 46514 Schermbeck-Gahlen (DE); GRIES, Thomas, 52072 Aachen (DE)
(74) Vertreter: Vossius & Partner Patentanwälte Rechtsanwälte mbB
(86) Internationale Anmeldenummer: PCT/EP2020/062432
(87) Internationale Veröffentlichungsnummer: WO 2020/225254

(56) Entgegenhaltungen:
- EP-A2- 1 433 440
- EP-A2- 1 693 009
- WO-A1-2009/025849
- US-A1- 2006 058 638
- US-A1- 2018 245 243
- LIBERSKI ALBERT ET AL: "Weaving for heart valve tissue engineering", BIOTECHNOLOGY ADVANCES, ELSEVIER PUBLISHING, BARKING, GB, vol. 35, no. 6, 4 August 2017 (2017-08-04), pages 633 - 656, XP085179351, ISSN: 0734-9750, DOI: 10.1016/J.BIOTECHADV.2017.07.012

## Beschreibung

Die vorliegende Erfindung betrifft ein Verfahren zur Herstellung eines Gefäßimplantats, insbesondere eines endoluminalen und/oder endovaskulären Gefäßimplantats, wobei das Gefäßimplantat ein oder mehrere Module mit jeweils mindestens einem tubulären Mantelkörper aufweist und wobei insbesondere ein computergestütztes Modell des Gefäßimplantats basierend auf erhaltenen Gefäßparametern erstellt wird und ein oder mehrere abgefragte und/oder eingegebene Strukturelemente eines jeweiligen Moduls in das computergestützte Modell integriert werden.

Gefäße sind für die Funktion des menschlichen Körpers von großer Bedeutung, da sie je nach Ausgestaltung Fluide transportieren und/oder temporär speichern können. Zahlreiche Faktoren wie beispielsweise Alterung, Ernährung, Stress oder Krankheit können mit der Zeit zu einer Veränderung der Gefäße, insbesondere der Blutgefäße, führen. Besonders risikobehaftet sind hierbei Änderungen in Bezug auf die Elastizität und die Stabilität der Gefäßwände, die sich oft auf den Durchmesser der Gefäße auswirken, sowie beispielsweise Änderungen in Bezug auf die Funktion von als Ventile wirkender Klappen.

Insbesondere lokale Veränderungen einer Gefäßwand sind nicht unüblich und können je nach betroffenem Gefäß und Lokalisation ausgesprochen kritisch für Patienten sein. Ein Beispiel für eine lokale Änderung der Gefäßwand stellen Aneurysmen dar, wobei verschiedene Definitionen des Begriffs "Aneurysma" Verwendung finden. So können beispielsweise Veränderungen einer Gefäßwand als Aneurysma bezeichnet werden, die zu einer Vergrößerung des Durchmessers des betroffenen Gefäßes führen, im Allgemeinen zu einer Vergrößerung um mindestens 50 % im Vergleich zu dem Durchmesser eines intakten Gefäßes in dem jeweiligen Bereich. Auch der absolute Durchmesser eines Gefäßes in dem Bereich der Gefäßwandveränderung kann als Indikation für ein Aneurysma herangezogen werden, beispielsweise im Falle einer abdominalen Aorta von 5 cm bis 5,5 cm bei Männern beziehungsweise von 4,5 cm bis 5 cm bei Frauen. Auch im Falle anderer Gefäße wie beispielsweise thorakaler oder thorako-abdominaler Aorten können entsprechende Werte, möglicherweise mit geringfügiger Abweichung, als Indikation für ein Aneurysma herangezogen werden. Gegebenenfalls kann die Zunahme des Gefäßdurchmessers auch geringer sein, insbesondere wenn die Veränderung vergleichsweise rasch voranschreitet. So kann beispielsweise die Zunahme des Gefäßdurchmessers von mindestens 5 mm innerhalb von 6 Monaten auf ein Aneurysma und damit auf ein erhöhtes Rupturrisiko des betroffenen Gefäßes hinweisen.

Gefäßimplantate stellen eine häufige genutzte Möglichkeit zur Verringerung der von Gefäßveränderungen ausgehenden Gefahr dar, beispielsweise durch Überbrücken oder Ersetzen des entsprechenden Gefäßabschnittes. So kann beispielsweise ein erweitertes Gefäß (etwa ein Gefäß mit einem Aneurysma gemäß einer der vorhergehenden Definitionen) durch einen Stentgraft überbrückt beziehungsweise ersetzt werden. Ein endovaskuläres Gefäßimplantat wie beispielweise ein Stentgraft kann somit den Blutfluss in dem jeweiligen Gefäß in einem klinisch unbedenklichen Maß sicherstellen und beispielsweise im Fall eines Aneurysmas einer Ruptur vorbeugen.

Die bereits genannten Stentgrafts stellen ein Beispiel für endovaskuläre Gefäßimplantate dar. Ein Stentgraft ist ein Hohlzylinder mit Längserstreckung, der zumindest einen tubulären Mantelkörper, den Graft, und eine Stentstruktur zur Stabilisierung aufweist. Stentgrafts können dabei modular aufgebaut sein. So kann beispielsweise der Graft mindestens einen Hauptmantelkörper und mindestens einen weiteren Mantelkörper umfassen, wobei die weiteren Mantelkörper gleiche oder vergleichbare oder auch verschiedene Durchmesser aufweisen können, und die Stentstruktur ein oder mehrere Stentstrukturelemente. Stentstruktur und Graft werden üblicherweise in getrennten Fertigungsprozessen hergestellt und anschließend assembliert, beispielsweise durch Vernähen. Beispiele für derartige Gefäßimplantate sind beispielsweise aus der GB 2 347 861 A, der US 2004/193258 A1 oder auch der US 5 911 753 A bekannt, und erhältliche Produkte finden bereits routinemäßig Anwendung.

Kommerziell erhältliche gewebte Grafts weisen verschiedene Webmuster auf (vergleiche Zhao et al, 2011, Adv Mat Res. 332-334:1498-1504). So werden beispielsweise Grafts von Cook Medical, Bloomington, USA, aus Multifilamentfäden hergestellt und weisen ein 1/1 planes Webmuster, auch Leinwandbindung genannt, auf. Grafts von Vascutek Limited, einer Terumo Gesellschaft, Inchinnan, Schottland, Großbritannien, werden hingegen aus Monofilamentfäden hergestellt und weisen ein 4/4 Twill-Webmuster auf.

Hinsichtlich der individuellen Gefäßgeometrie eines jeden Patienten sind neben klinisch etablierten Standardprodukten patientenindividualisierte Lösungen wünschenswert, wenn nicht sogar notwendig. Diese sind allerdings noch immer mit vergleichsweise hohen Verkaufspreisen und vielen sowie zeitintensiven Produktionsschritten verbunden. Patientenspezifische medizinische Bilddaten wie Daten aus CT Verfahren können bereits mittels kommerziell erhältlicher Computerprogramme dreidimensional dargestellt und bearbeitet werden, beispielsweise mit Programmen wie Aquarius iNtuition EVAR (Vessel Analysis) Planning von TeraRecon, Inc., Foster City, Kalifornien, USA, oder auch dem Programm 3mensio Vascular von Pie Medical Imaging BV, Maastricht, Niederlande. Derartige Computerprogramme bieten jedoch keine oder keine effiziente Übersetzung der Bilddaten in Formate, die von Maschinen zur Herstellung von Gefäßimplantaten genutzt werden können, insbesondere nicht von textilen und/oder textilbasierten Gefäßimplantaten wie Grafts. Zudem fehlt es an entsprechend benötigten Schnittstellen zur Datenübergabe.

Ein Verfahren zur Erstellung eines individualisierten Stentgrafts basierend auf einem digitalen 3D-Modell ist beispielsweise aus der DE 10 2015 207 596 A1 bekannt. Dabei werden jedoch eine Vielzahl von Kunststoff- oder Kunstharzschichten mittels eines additiven Verfahrens aufeinander aufgetragen. Dass den vielfältigen klinischen Anforderungen an eine solche endovaskuläre Gefäßstütze auf diese Weise genügt werden kann, scheint jedoch fraglich.

Ein weiteres Verfahren zur Herstellung eines individualisierten Gefäßimplantats ist aus der US 2019/0050507 A bekannt. Hierbei wird ein Modell eines Gefäßes erzeugt, anhand dessen ein Standard-Gefäßimplantat modifiziert wird.

Vor diesem Hintergrund beabsichtigt die vorliegende Erfindung, ein automatisierbares, zeit- und kosteneffizientes Verfahren bereit zu stellen, das die Herstellung von individualisierten Gefäßimplantaten, insbesondere von individualisierten endovaskulären Gefäßimplantaten, vereinfacht. Die Erfindung beabsichtigt zudem, dass die hergestellten Gefäßimplantate eine hohe Biokompatibilität aufweisen und den Anforderungen in der klinischen Praxis genügen. Hierzu zählen insbesondere die Berücksichtigung patientenspezifischer anatomischer Besonderheiten in dem jeweiligen von dem Implantat zu unterstützenden und/oder zu überbrückendem Abschnitt des jeweiligen Gefäßes.

Erfindungsgemäß wird dies durch ein Verfahren und ein Gefäßimplantat, insbesondere ein endovaskuläres Gefäßimplantat, gemäß den unabhängigen Ansprüchen erreicht, wobei vorteilhafte Ausgestaltungen mit zweckmäßigen Weiterbildungen der Erfindung in den jeweiligen abhängigen Ansprüchen aufgeführt sind. Vorteilhafte Ausgestaltungen eines Erfindungsaspekts sind dabei als wechselseitig vorteilhafte Ausgestaltungen des jeweiligen anderen Erfindungsaspektes anzusehen.

Aus der WO 2009/025849 A1 sind Verfahren und Vorrichtungen bekannt, bei denen eine oder mehrere Blutgefäßschlauchdimensionen basierend auf Blutgefäßdaten eines Individuums akzeptiert werden. Basierend auf der einen oder den mehreren Blutgefäßschlauchdimensionen wird durch Rapid-Prototyping ein Blutgefäßschlauch hergestellt.

Ein erster Aspekt der Erfindung betrifft ein Verfahren zur Herstellung eines insbesondere endovaskulären Gefäßimplantats, das die folgenden Schritte aufweist: Erhalten von Gefäßparametern; Erstellen eines computergestützten Modells eines Gefäßimplantats, wobei das Gefäßimplantat ein oder mehrere Module mit jeweils mindestens einem tubulären Mantelkörper aufweist, basierend auf den erhaltenen Gefäßparametern; Auswählen eines oder mehrerer Strukturelemente eines jeweiligen Moduls aus der Gruppe bestehend aus: einer oder mehreren Durchmesseränderungen entlang mindestens eines tubulären Mantelkörpers, einer oder mehreren Bifurkationen, einer oder mehreren Abzweigungen, einer oder mehreren Aussparungen, einer oder mehreren lokalen Verstärkungen; Bestimmen von Parametern zu dem einen oder den mehreren ausgewählten Strukturelementen; Integrieren der Strukturelemente gemäß den bestimmten Parametern in das computergestützte Modell; und Herstellen des Gefäßimplantats anhand des erstellten computergestützten Modells. Das Gefäßimplantat ist textil und/oder textilbasiert, vorzugsweise ein Gewebe und/oder eine Maschenware. Insbesondere kann das Gefäßimplantat mittels Jacquard-Technik hergestellt sein. Unter dem "Auswählen" eines Strukturelements ist eine manuelle, teilautomatische oder vollautomatische Auswahl zu verstehen. Eine manuelle oder teilautomatische Auswahl kann eine Abfrage zur Eingabe des oder der gewünschten Strukturelemente durch einen Nutzer umfassen. Das Herstellen des Gefäßimplantats anhand des erstellten computergestützten Modells umfasst insbesondere das Wirken des Gefäßimplantats anhand dieses Modells, insbesondere das Wirken mittels Jacquard-Technik.

Das erfindungsgemäße Verfahren zur Herstellung eines, ein oder mehrere Module mit jeweils mindestens einem tubulären Mantelkörper aufweisendem, insbesondere endovaskulären, Gefäßimplantats weist somit insbesondere das Erstellen eines computergestützten Modells des Gefäßimplantats basierend auf jeweilig erhaltenen patientenspezifischen Gefäßparametern auf, in das nach jeweiliger Abfrage ein oder mehrere Strukturelemente integriert werden und das daraufhin zur Herstellung des textilen und/oder textilbasierten Gefäßimplantats verwendet wird.

Im Kontext der vorliegenden Erfindung bezieht sich der Begriff "Gefäßimplantat" auf ein Implantat oder auch eine Prothese, das in ein Gefäß, insbesondere ein Blutgefäß, eingebracht werden kann, um einen jeweiligen Gefäßabschnitt zu überbrücken oder auch zu ersetzen. Das Gefäßimplantat kann auch einer Anastomose an ein (erkranktes oder ansonsten auch gesundes) Gefäß dienen, insbesondere an ein (erkranktes oder gesundes) Blutgefäß. Ein erfindungsgemäßes Gefäßimplantat weist dabei ein oder mehrere Module mit jeweils mindestens einem tubulären Mantelkörper auf. Ein erfindungsgemäßes Gefäßimplantat, z.B. ein erfindungsgemäßes endovaskuläres Gefäßimplantat, kann ferner dazu ausgelegt sein, in ein einzelnes Gefäß eingebracht zu werden oder auch sich in mehr als einem Gefäß zu erstrecken. Letzteres ist beispielsweise vorteilhaft, wenn sich der zu ersetzende oder zu überbrückende Abschnitt an oder nahe einer Verzweigung des Gefäßes befindet. Der im Folgenden verwendete Begriff "Gefäßimplantat" ist synonym zu verstehen, sofern nichts Gegenteiliges angegeben ist.

Im Kontext der vorliegenden Erfindung bezeichnet der Begriff "Gefäß" jede Art tubulärer Hohlkörper, insbesondere des menschlichen Körpers, der mindestens ein Fluid wie beispielsweise ein Gas und/oder eine Flüssigkeit transportieren und/oder speichern kann. Der Begriff bezeichnet nicht ausschließlich, aber insbesondere Hohlgefäße wie beispielsweise Blutgefäße. Das Gefäß kann in verschiedenen Körperregionen lokalisiert sein, beispielsweise in den Extremitäten oder im Gehirn, insbesondere aber im Abdomen und/oder Thorax.

Als "zu überbrückender Gefäßabschnitt", "zu ersetzender Gefäßabschnitt" oder auch als "ein Gefäßabschnitt, für den ein Gefäßimplantat, etwa ein endovaskuläres Gefäßimplantat, hergestellt werden soll" wird im Folgenden insbesondere ein Abschnitt eines Gefäßes mit einer genetisch bedingten und/oder erworbenen Gefäßwandveränderung bezeichnet, der ausgekleidet, überbrückt und/oder ersetzt werden soll. Die Ausdrücke sind synonym zu verstehen und beziehen sich auf den lokal begrenzten Abschnitt des jeweiligen Gefäßes mit der Gefäßwandveränderung sowie die unmittelbar daran angrenzenden Abschnitte. Zur Verdeutlichung kann letzteres auch zusätzlich noch mit angegeben sein, beispielsweise als "der zu ersetzende Gefäßabschnitt und die unmittelbar daran angrenzenden Gefäßabschnitte" oder in Form von vergleichbaren Ausdrücken. Darüber hinaus kann das Gefäßimplantat auch in Gefäßen oder Gefäßbereichen eingesetzt werden, in denen keine Gefäßwandveränderung oder -schwächung vorliegt, etwa falls eine Anastomose in einem gesunden Gefäßwandbereich hergestellt werden soll.

Im Kontext der vorliegenden Erfindung bezeichnet der Begriff "Gefäßparameter" Zustände, Geometrie und/oder Eigenschaften, die ein Gefäß in dem jeweiligen zu überbrückenden und/oder zu ersetzenden Abschnitt i) aufweist, ii) aufweisen würde, wenn keine Gefäßwandveränderung vorläge unter Berücksichtigung verschiedener Faktoren, wie beispielsweise Alter, Geschlecht und/oder Körpergewicht, und/oder iii) aufgewiesen hat, bevor eine jeweilige Gefäßwandveränderung entstand. Der Begriff kann sich auf den Abschnitt des Gefäßes mit der Gefäßwandänderung beziehen und/oder auch Zustände, Geometrie und/oder Eigenschaften der unmittelbar angrenzenden Gefäßabschnitte umfassen. Gefäßparameter können geometrische Parameter sowie optional auch morphologische Parameter umfassen. Geometrische Parameter können beispielsweise Parameter zu Gefäßverläufen sein, Durchmesser, Formen des Querschnitts und/oder deren Veränderung, Längen, Winkel und/oder Positionen des jeweiligen Gefäßabschnittes, etwa eines zu ersetzenden Gefäßabschnittes. Morphologische Parameter können beispielsweise Parameter sein, die sich auf das Vorhandensein von Kalzifizierung und/oder Thrombus beziehen und/oder die den Grad der Kalzifizierung, die Menge an Thrombus und/oder die Ausdehnung des von einem jeweiligen Grad der Kalzifizierung und/oder Menge an Thrombus betroffenen Bereichs im jeweilig zu ersetzenden Gefäßabschnitt und/oder in unmittelbar daran angrenzenden Gefäßabschnitten angeben.

Gemäß dem erfindungsgemäßen Verfahren werden zunächst Gefäßparameter bezüglich des jeweilig zu überbrückenden Gefäßabschnittes erhalten. Diese beziehen sich insbesondere auf Zustände, Geometrie und/oder Eigenschaften, die ein Gefäß in dem zu überbrückenden und/oder zu ersetzenden Abschnitt aufweist. Die erhaltenen Gefäßparameter enthalten bevorzugt patientenspezifische Informationen bezüglich Durchmesser, Durchmesseränderungen und/oder Länge des zu ersetzenden Gefäßabschnitts. Alternativ oder zusätzlich können die Gefäßparameter Angaben zu Verzweigungen in dem zu ersetzenden Gefäßabschnitt umfassen, insbesondere zu Bifurkationen und/oder Abzweigungen. Unter einer "Bifurkation" wird im Rahmen der vorliegenden Anmeldung die Aufspaltung des Gefäßes in zwei Gefäße mit ähnlichem Durchmesser verstanden (etwa die Bifurkation der Aorta in die beiden Beckenarterien). Unter einer "Abzweigung" wird im Rahmen der vorliegenden Anmeldung ein oder mehrere von einem Hauptgefäß abgehende Nebengefäße kleineren Durchmessers verstanden, etwa die von der Aorta abzweigenden Nierenarterien, die Abgänge im Bereich des Aortenbogens oder die Herzkranzgefäße. Durch das Erhalten patientenspezifischer Gefäßparameter kann eine optimale Passform des herzustellenden Gefäßimplantats für den zu ersetzenden Gefäßabschnitt sichergestellt werden und das Risiko einer mangelhaften, beispielsweise unvollständigen, Überbrückung und/oder Ersetzung des jeweiligen Gefäßabschnitts minimiert werden. Weitere Angaben zu den erhaltenen Gefäßparametern sind der nachfolgenden Beschreibung der Schritte bezüglich des Erhaltens und Visualisierens von Bilddaten eines Gefäßes, des Identifizierens des Gefäßabschnittes, für den ein Gefäßimplantat (z.B. ein endovaskuläres Gefäßimplantat) hergestellt werden soll und des Messens von jeweiligen Gefäßparametern zu entnehmen. Der Gefäßabschnitt kann insbesondere ein Abschnitt mit einem Aneurysma sein.

Die Gefäßparameter können basierend auf einem durch ein bildgebendes Verfahren gewonnenen Datensatz zumindest des zu ersetzenden Gefäßabschnittes sowie der unmittelbar daran angrenzenden Gefäßabschnitte erhalten werden. Das Aufbereiten eines solchen Datensatzes und das Erhalten von jeweiligen Gefäßparametern, beispielsweise durch Vermessen, umfassen dabei dem Fachmann bekannte Schritte, wie sie beispielsweise in 3mensio Vascular oder vergleichbaren Computerprogrammen implementiert sind. Die Gefäßparameter beziehungsweise entsprechende Daten, die mittels eines bildgebenden Verfahrens erhalten wurden, können im Rahmen des Verfahrens von einem Speichermedium (etwa von einer Festplatte) ausgelesen oder elektronisch übertragen werden,. Die erhaltenen Gefäßparameter werden bevorzugt in einem von einem jeweiligen Computerprogramm lesbaren Format erhalten und zur Erstellung eines computergestützten Modells eines Gefäßimplantats herangezogen. Bevorzugt wird eine Fehlermeldung für den Anwender ausgegeben, wenn die Gefäßparameter nicht in einem von dem jeweiligen Computerprogramm lesbaren Format erhalten werden und/oder unvollständig sind, beispielsweise auf Grund eines Übertragungsfehlers.

Gemäß dem erfindungsgemäßen Verfahren wird ein computergestütztes Modell eines Gefäßimplantats mit einem tubulären Mantelkörper basierend auf den erhaltenen Gefäßparametern erstellt, bevorzugt mittels eines Computerprogramms. Das computergestützte Modell kann beispielsweise ein digitales 3D-Modell sein. Das computergestützte Modell kann auch nur temporär ein digitales 3D-Modell sein, das in andere Modelle umgewandelt wird und/oder von anderen Modellen in ein digitales 3D-Modell umgewandelt wird. Beispielsweise kann anhand eines digitalen 3D-Modells eines Gefäßimplantats (z.B. eines endovaskulären Gefäßimplantats) eine technische Zeichnung in 2D erstellt werden. Dies erfolgt bevorzugt durch dasselbe Computerprogramm, das bereits zur Erstellung des 3D-Modells verwendet wurde, kann jedoch auch durch ein weiteres Computerprogramm erfolgen. Eine technische 2D-Zeichnung stellt ein geometrisches Modell des Gefäßimplantats (z.B. des endovaskulären Gefäßimplantats) dar, das in Herstellungsinformationen für eine jeweilige Produktionsmaschine übersetzt werden kann, bevorzugt in eine maschinenlesbare Datei, insbesondere eine KMO Datei. Alternativ kann eine KMO Datei direkt aus dem computergestützten 3D-Modell erstellt werden. Der Vorteil einer KMO Datei besteht darin, dass diese direkt in eine jeweilige Textilmaschine eingelesen werden kann und die in der Datei enthaltenen Informationen insbesondere mittels einer Wirkmaschine umgesetzt werden können.

Eine KMO Datei, oder eine vergleichbare Datei, kann beispielsweise aus dem erstellten 3D-Modell abgeleitet werden (z.B. direkt) oder auch aus einer, die Position jeder einzelnen Nadel in dem jeweiligen Maschenbildungsprozess beinhaltenden Pixeldatei, insbesondere einer JC Datei, erstellt werden. Eine JC Datei wiederum kann direkt aus dem erstellten 3D-Modell abgeleitet werden oder aus einer Designdatei erstellt werden, etwa einer farb-codierten 2D-Pixeldatei. So kann beispielsweise basierend auf den in Form eines 3D-Modells oder einer technischen Zeichnung bereitgestellten Daten und/oder Parametern eine farb-codierte 2D-Pixeldatei erstellt werden, wobei die Farben der Pixel jeweilige Nadelstellungen codieren, die zur Herstellung des Gefäßimplantats benötigt werden. Anhand einer jeweiligen farb-codierten 2D-Pixeldatei kann beispielsweise in Kombination mit Informationen zur Legung (Barrenbewegung im Wirkprozess) - die beispielsweise in Form einer Legungsdatei erhalten werden, die die Legung und somit die daraus resultierende Bindung für jeweilige Legebarren beinhaltet - eine jeweilig überwiegend verwendete Bindung sowie Änderungen hierzu, wie beispielsweise jeweilige Bindungen zur Einbringung von Aussparungen, lokalen Verstärkungen, und/oder Trennfäden, bestimmt werden. Die farb-codierte 2D-Pixeldatei kann in eine JC Datei umgewandelt werden, wobei jede Farbe in der farb-codierten 2D-Pixeldatei für eine Abfolge von z.B. acht, bevorzugt binären, Maschinenbefehlen steht. Die JC Datei ist dabei vorzugsweise eine 2D-Pixeldatei, welche in, bevorzugt binär codierten, Pixeln verschiedene Nadelpositionen (z.B. "hoch" und "tief") codiert. Eine JC Datei kann somit beispielsweise rote und weiße Pixel umfassen. Eine JC-Datei kann wiederum in eine von einer Wirkmaschine lesbare Datei, die Informationen zur Herstellung eines jeweiligen Gefäßimplantats enthält, insbesondere zu Nadelpositionen und damit zur Bindung, umgewandelt werden, bevorzugt in eine KMO Datei. Das jeweilige digitale 3D-Modell, die jeweilige technische Zeichnung und/oder die jeweilige Datei, etwa Designdatei, JC Datei und/oder KMO Datei, wird bevorzugt abgespeichert oder zumindest zwischengespeichert, etwa auf einem Speichermedium. Die Erstellung einer farb-codierten 2D-Pixeldatei, die Übertragung jeweiliger Informationen in eine JC Datei und die Erstellung einer KMO Datei aus einer jeweiligen JC Datei kann beispielsweise mittels eines Computerprogrammes erfolgen, wie beispielweise DesignScope Jacquard Raschel, EAT GmbH, Krefeld, Deutschland.

Im Kontext der vorliegenden Erfindung umfasst der Begriff "computergestütztes Modell" somit ein oder mehrere digitale 2D-Modelle, ein oder mehrere digitale 3D-Modelle, ein oder mehrere technische Zeichnungen, ein oder mehrere Designdateien, ein oder mehrere JC Dateien sowie ein oder mehrere KMO Dateien. Bevorzugt umfasst das computergestützte Modell ein 3D-Modell und/oder eine maschinenlesbare Datei, insbesondere eine KMO Datei, wobei der Schritt des Herstellens des Gefäßimplantats vorzugsweise auf Basis der maschinenlesbaren Datei erfolgt. Dies hat den Vorteil, dass die für die Herstellung des Gefäßimplantats benötigten Parameter in einem Format vorliegen, das von Maschinen zur Herstellung insbesondere textiler und/oder textilbasierter Gefäßimplantate direkt gelesen und verarbeitet werden kann.

Das erfindungsgemäße Verfahren kann ferner einen Schritt der Visualisierung des computergestützten Modells des Gefäßimplantats umfassen. Eine Visualisierung des Modells ermöglicht eine optische Kontrolle und kann dadurch die Kontrolle des Verfahrens während des Herstellungsprozesses erleichtern und/oder beschleunigen. So können beispielsweise frühzeitig fehlende und/oder unvollständige Datensätze ermittelt werden und der Erstellung eines unvollständigen und/oder mangelhaften Gefäßimplantats vorgebeugt werden. Sollten beispielsweise die erhaltenen Gefäßparameter nur einen Teil des zu ersetzenden oder zu überbrückenden Gefäßabschnittes und/oder der unmittelbar angrenzenden Gefäßabschnitte abdecken, so kann in dem erfindungsgemäßen Verfahren eine Ausgabe eines jeweiligen Hinweises für den Anwender vorgesehen sein. Bevorzugt erfolgt nach einer jeweiligen Visualisierung eine Abfrage, ob der Anwender nach einer optischen Kontrolle zustimmt mit dem erfindungsgemäßen Verfahren fortzufahren. Ferner ermöglicht eine Visualisierung des Modells eine Simulation des Implantats und/oder von dessen Implantation im Gefäßabschnitt. Alternativ oder zusätzlich kann hierdurch auch eine Simulation des Verhaltens des Gefäßimplantats unter mechanischer Belastung beispielsweise während und/oder nach einer Implantation bereitgestellt werden. Ferner können basierend auf der optischen Kontrolle und/oder Simulation des Gefäßimplantats Änderungen an einem oder mehreren Parametern des computergestützten Modells vorgenommen werden, z.B. an einem oder mehreren geometrischen Parametern wie dem Durchmesser und/oder der Länge von einem oder mehreren Abschnitten und/oder von Winkeln zwischen Abschnitten.

Das erfindungsgemäße Verfahren weist ferner die Schritte des Auswählens eines oder mehrerer Strukturelemente eines jeweiligen Moduls auf, des Bestimmens von Parametern zu dem oder den mehreren ausgewählten Strukturelementen, und des Integrierens der Strukturelemente gemäß den bestimmten Parametern in das computergestützte Modell. Dies hat den Vorteil, dass das Gefäßimplantat optimal an das jeweilige zu ersetzende Gefäß angepasst werden kann und entsprechend den jeweiligen patientenspezifischen und positionsspezifischen Belastungen ausgestaltet werden kann. Dies hat einen positiven Effekt auf die Lebensdauer und die Funktionalität des Gefäßimplantats. Je nach Ausgestaltung des Verfahrens kann die Abfrage manuell oder auch automatisch erfolgen. Die Parameter können durch einen Nutzer bestimmt werden und/oder automatisch aus den erhaltenen Gefäßparametern erzeugt werden.

Das mindestens eine Strukturelement ist hierbei ausgewählt aus der Gruppe bestehend aus: einer oder mehreren Durchmesseränderungen entlang mindestens eines tubulären Mantelkörpers, einer oder mehreren Bifurkationen, einer oder mehreren Abzweigungen, einer oder mehreren Aussparungen, einer oder mehreren lokalen Verstärkungen, und einem oder mehreren Iliakalgefäß-Grafts. Das mindestens eine Strukturelement wird bevorzugt patientenindividuell ausgestaltet und in das jeweilige computergestützte Modell des Gefäßimplantats integriert. Auf diese Weise kann eine optimale Passform des Gefäßimplantats bezüglich des zu ersetzenden oder überbrückenden Gefäßabschnitts sowie optional der unmittelbar daran angrenzenden Gefäßabschnitte sichergestellt werden. Dies hat einen positiven Effekt auf Funktionalität und Lebensdauer des Gefäßimplantats, insbesondere der Dauerfestigkeit, nach dessen Einbringen in den jeweiligen zu überbrückenden Gefäßabschnitt. Je nach den aus der Gestalt des Gefäßes resultierenden Anforderungen kann das Gefäßimplantat mindestens zwei der genannten Strukturelemente aufweisen, etwa mindestens eine Bifurkation und eine oder mehrere Abzweigungen, mindestens eine Bifurkation und eine oder mehrere Aussparungen (optional mit einer oder mehreren lokalen Verstärkungen an diesen Aussparungen), mindestens eine Durchmesseränderung und eine oder mehrere Abzweigungen, mindestens eine Durchmesseränderung und eine oder mehrere Aussparungen (optional mit einer oder mehreren lokalen Verstärkungen an diesen Aussparungen), etc.

Eine Durchmesseränderung entlang mindestens eines tubulären Mantelkörpers eines jeweiligen Moduls kann insbesondere bei größeren Gefäßimplantaten in Bezug auf eine optimale Positionierung und maximale Funktionsfähigkeit vorteilhaft sein. Beispielsweise kann, insbesondere in Bereichen, die unmittelbar an den lokal begrenzten Abschnitt eines jeweiligen Gefäßes mit Gefäßwandveränderung angrenzen und den Bereich des Kontakts zwischen Gefäßimplantat und Gefäßwand darstellen, ein sogenanntes Oversizing vorteilhaft sein. Im Falle eines Oversizings kann das Gefäßimplantat beispielsweise einen 10 % bis 20 % größeren Durchmesser als das Gefäß im jeweiligen Bereich des Kontakts zwischen Gefäßimplantat und Gefäßwand aufweisen. Durch das Oversizing kann eine definierte Radialkraft vom Gefäßimplantat auf die Gefäßwand im jeweiligen Bereich des Kontakts zwischen Gefäßimplantat und Gefäßwand wirken, wodurch eine Abdichtung sowie eine lagefeste Positionierung des Gefäßimplantats in dem zu überbrückenden Gefäßabschnitt gewährleistet werden kann.

Ferner kann mindestens eine Durchmesseränderung entlang eines jeweiligen tubulären Mantelkörpers vorteilhaft sein, wenn der jeweilig zu ersetzende Gefäßabschnitt mindestens eine Durchmesseränderung in Strömungsrichtung des Fluids aufweist. Wäre der Durchmesser des Gefäßimplantats an den kleinsten Durchmesser des zu ersetzenden Gefäßabschnittes angepasst, so könnten Abdichtung und lagefeste Positionierung des Gefäßimplantats in jeweiligen, direkt an den Bereich des zu ersetzenden Gefäßabschnitts angrenzenden Bereichen mit größeren Durchmessern beeinträchtigt werden. Weiterhin könnte entweder das zu transportierende oder zu speichernde Fluid sich in dem oder den zwischen Gefäßwand und Gefäßimplantat entstehenden Zwischenräumen stauen und/oder verwirbeln und so für einen lokal erhöhten Druck auf die Gefäßwand sorgen. Würde hingegen der Durchmesser des Gefäßimplantats an den größten Durchmesser des zu ersetzenden Gefäßabschnittes angepasst und dies nicht im Rahmen eines Oversizings geschehen, so könnte dies für einen, sich gegebenenfalls nachteilig auswirkenden, lokal erhöhten Druck auf die Gefäßwand sorgen und zwar in Bereichen, in denen das jeweilige Gefäß einen kleineren Durchmesser aufweist als das einzusetzende Gefäßimplantat. Würde der Durchmesser des Gefäßimplantats an den größten Durchmesser des zu ersetzenden Gefäßabschnittes angepasst, so könnte alternativ oder zusätzlich das Problem auftreten, dass das Implantat nicht optimal positioniert werden kann und beispielsweise Falten schlägt, wodurch die Dichtigkeit beeinträchtigt werden kann und/oder eine Druckerhöhung durch Verringerung des Durchmessers entstehen kann, beispielsweise durch eine durch die Faltung bedingte Stauung des Blutflusses und/oder Thrombusbildung. Eine derartige Thrombusbildung kann insbesondere im Bereich der sogenannten Totzonen der Falten auftreten und ein nicht unerhebliches Risiko darstellen, insbesondere, wenn sich der Thrombus löst. Solche Problematiken können insbesondere bei längeren Gefäßimplantaten auftreten, wie beispielsweise bei thorako-abdominalen Aneurysmen. Das Gefäßimplantat kann eine Länge in einer jeweiligen Haupterstreckungsrichtung (insbesondere in Längsrichtung entlang des Gefäßes) von mehr als 3 cm, bevorzugt von mindestens 5 cm, besonders bevorzugt von mindestens 7 cm aufweisen, beispielsweise zur Behandlung derartiger Aneurysmen.

Die Bereitstellung mindestens einer Durchmesseränderung kann auch im Bereich eines oder mehrerer Iliakalgefäß-Grafts von Interesse sein. So kann der Durchmesser eines Hauptmantelkörpers am Ende einer Bifurkation (z.B. am Ende der Bifurkation in Strömungsrichtung) größer oder auch kleiner als der Durchmesser des jeweiligen Iliakalgefäßes sein. Indem sich der Iliakalgefäß-Graft verjüngt (also bei Einsatz des Gefäßimplantats in der Aorta in Strömungsrichtung des Bluts verjüngt), kann der Durchmesser des Hauptmantelkörpers (z.B. der Durchmesser am Hauptmantelkörper am Ende der Bifurkation) auf den Durchmesser des Iliakalgefäßes angepasst werden.

Durchmesseränderungen entlang eines oder mehrerer Module des Gefäßimplantats können zudem auch herstellungsbedingt günstig sein, um den modularen Aufbau zu erleichtern. So kann ein jeweiliges Modul an einem ersten Ende an den Durchmesser des Gefäßes und an einem zweiten Ende an den Durchmesser eines vorangehenden oder nachfolgenden Moduls angepasst sein. Zudem kann ein jeweiliges Modul an einem ersten Ende an den Durchmesser eines vorangehenden Moduls und an einem zweiten Ende an den Durchmesser eines nachfolgenden Moduls angepasst sein.

Im Zuge des Verfahrens kann bestimmt werden, ob eine Durchmesseränderung wünschenswert ist, beispielsweise indem die Durchmesser des herzustellenden Gefäßimplantats an den Enden des mindestens einen tubulären Mantelkörpers und/oder an verschiedenen Positionen innerhalb des mindestens einen tubulären Mantelkörpers verglichen werden, beispielsweise anhand der erhaltenen Gefäßparameter. Ergibt sich hierbei eine Abweichung von mindestens 5 mm, bevorzugt von mindestens 3 mm, besonders bevorzugt von mindestens 2 mm kann dies als Kriterium dafür herangezogen werden, dass eine Durchmesseränderung wünschenswert ist und eine solche Durchmesseränderung kann beispielsweise in das computergestützte Modell des Gefäßimplantats integriert werden. Dies kann insbesondere durch eine gleichmäßige Änderung des Durchmessers in dem jeweiligen Bereich des Gefäßimplantats erfolgen oder auch durch eine stufenartige beziehungsweise sprungweise Änderung des Durchmessers. Auch Kombinationen dieser beiden Möglichkeiten sind denkbar für eine besonders gute Passform des Gefäßimplantats. Eine Durchmesseränderung kann z.B. durch eine Reduktion oder Erhöhung der verwendeten Fadenanzahl, das heißt durch eine Reduktion oder Erhöhung der Maschenstäbchenzahl und/oder durch eine Erhöhung oder Reduktion der Maschendichte erreicht werden. Alternativ oder zusätzlich kann eine Durchmesseränderung nach dem Herstellen des Gefäßimplantats insbesondere durch einen Schritt des Thermoformens und/oder Thermofixierens des Gefäßimplantats auf einem Kern erreicht werden.

Ferner kann es vorteilhaft sein, eine oder mehrere Bifurkationen und/oder Abzweigungen entlang des mindestes einen tubulären Mantelkörpers des Gefäßimplantats vorzusehen. Dies ist besonders vorteilhaft, wenn der zu ersetzende Gefäßabschnitt selbst eine Verzweigung und/oder eine Verzweigung in unmittelbarer Nähe aufweist und/oder eine künstliche Öffnung zum Zweck einer Anastomose hergestellt werden soll. Indem das Gefäßimplantat ebenfalls eine jeweilige Verzweigung aufweist, kann eine optimale und lagefeste Positionierung des Gefäßimplantats erreicht werden. Ferner kann durch ein Gefäßimplantat, das wie der jeweilig zu ersetzende Gefäßabschnitt ebenfalls eine Verzweigung aufweist, die jeweilige Funktion des zu ersetzenden Gefäßabschnitts sowie der unmittelbar daran angrenzenden Bereiche, insbesondere ein Transport von Blut, nach einer Implantation des Gefäßimplantats wiederhergestellt und/oder sichergestellt werden.

Als "Bifurkation" des Gefäßimplantats wird im Kontext der vorliegenden Erfindung eine Verzweigung an einem der Enden des Gefäßimplantats bezeichnet, insbesondere eine Aufspaltung des Gefäßimplantats, bevorzugt in zwei tubuläre Mantelkörper mit insbesondere am Aufspaltungspunkt und/oder in dessen unmittelbarer Nähe vergleichbaren oder ähnlichen Durchmessern. Die beiden durch eine Bifurkation resultierenden Bifurkationsmantelkörper weisen somit zumindest am Aufspaltungspunkt und/oder in dessen unmittelbarer Nähe jeweilige Durchmesser auf, die sich beispielsweise um maximal 15 %, bevorzugt um maximal 10 %, besonders bevorzugt um maximal 5 % bezogen auf den jeweilig größeren Durchmesser voneinander unterscheiden. Die beiden tubulären Mantelkörper sind vorzugsweise einstückig mit dem Gefäßimplantat hergestellt.

Als "Abzweigung" oder auch "Branch" des Gefäßimplantats wird hingegen eine Verzweigung entlang des mindestens einen tubulären Mantelkörpers des Gefäßimplantats bezeichnet, wobei der in der Haupterstreckungsrichtung des Gefäßimplantats vorgesehene beziehungsweise vorliegende tubuläre Mantelkörper im Folgenden auch zu Verdeutlichung als Hauptmantelkörper bezeichnet werden kann und ein von diesem abzweigender tubulärer Mantelkörper als Seitmantelkörper. Der Seitmantelkörper weist hierbei einen deutlich geringeren Durchmesser als der Hauptmantelkörper am Aufspaltungspunkt und/oder in dessen unmittelbarer Nähe auf, beispielsweise einen um mindestens 25 % reduzierten Durchmesser, bevorzugt einen um mindestens 50 %, besonders bevorzugt einen um mindestens 75 % reduzierten Durchmesser.

Eine Aussparung ist erfindungsgemäß ebenfalls entlang des mindestens einen tubulären Mantelkörpers des Gefäßimplantats vorgesehen. Bei einer solchen Aussparung kann es sich insbesondere um eine Fenestrierung und/oder einen Scallop handeln. Eine Aussparung ist besonders vorteilhaft, wenn der zu ersetzende Gefäßabschnitt selbst eine Verzweigung und/oder eine Verzweigung in unmittelbarer Nähe aufweist. In diesem Fall können statt Verzweigungen des Gefäßimplantats wie Bifurkationen und/oder Abzweigungen entsprechende jeweilige Aussparungen vorgesehen werden, die eine schnellere und kostengünstigere Produktion erlauben.

Der Begriff "Fenestrierung" bezeichnet im Kontext der vorliegenden Erfindung Fenster, offene Zonen oder Löcher im Gefäßimplantat, die bei korrekter Positionierung des Gefäßimplantats dazu ausgebildet sind, in dem jeweiligen zu ersetzenden Gefäßabschnitt hinsichtlich Position und Durchmesser mit einem jeweiligen abzweigenden Gefäß in etwa, bevorzugt gänzlich, deckungsgleich zu sein.

Als "Scallop" wird im Kontext der vorliegenden Erfindung hingegen eine Aussparung an einem Ende des mindestens einen tubulären Mantelkörpers bezeichnet. Bevorzugt bezeichnet der Begriff Scallop hierbei eine dreieckige, viereckige, halbkreisförmige, ovale oder U-förmige Aussparung am, bevorzugt in Strömungsrichtung aufwärts gelegenen, Ende des Gefäßimplantats. Ein Scallop ist folglich zum jeweiligen Ende des Gefäßimplantats hin offen, insbesondere in Haupterstreckungsrichtung des jeweiligen tubulären Mantelkörpers des jeweiligen Moduls.

Ferner umfassen Strukturelemente auch eine oder mehrere lokale Verstärkungen. Der Begriff "lokale Verstärkung" bezeichnet im Kontext der vorliegenden Erfindung lokal in die Textilstruktur eingebrachte oder einzubringende Verstärkungsstrukturen. Sie sind insofern besonders vorteilhaft, als dass sie eine lastgerechte Ausgestaltung des Gefäßimplantats ermöglichen. Lokale Verstärkungen können lokal in die Textilstruktur des Gefäßimplantats eingebrachte Materialien umfassen, wie beispielsweise Garne und/oder Drähte, und/oder Verstärkerstrukturen, welche jeweils beispielsweise aus einem oder mehreren Garnen und/oder Drähten bestehen oder umfassen. Optional oder alternativ können lokale Verstärkungen auch lokal auf die Textilstruktur aufgebrachte Materialien umfassen, wie beispielsweise mindestens eine polymere Struktur, etwa aus Silikon. Lokale Verstärkungen sind besonders bevorzugt als lokale Änderungen der Bindung ausgebildet, insbesondere um eine lastgerechte Auslegung der Textilstruktur zu ermöglichen. Dadurch kann auch ein lastgerecht ausgestaltetes textiles und/oder textilbasiertes Gefäßimplantat einstückig gewirkt werden, wodurch es besonders widerstandsfähig gestaltet werden kann. Lokale Verstärkungen können auch ausgebildet sein als eine Kombination aus lokal in die Textilstruktur des Gefäßimplantats eingebrachte Materialien, lokal auf die Textilstruktur aufgebrachte Materialien, und/oder Änderungen der Bindung. Lokale Verstärkungen können in der textilen Fläche des Gefäßimplantats vorgesehen sein und/oder sich darin befinden und/oder am Übergang der textilen Fläche des Gefäßimplantats zu einem jeweiligen Strukturelement ausgewählt aus der Gruppe bestehend aus Durchmesseränderung entlang mindestens eines tubulären Mantelkörpers eines jeweiligen Moduls, Bifurkation, Abzweigung, und Aussparung. Somit können lokale Verstärkungen allein und/oder in Verbindung mit einem oder mehreren der zuvor genannten Strukturelemente in ein Gefäßimplantat integriert werden.

Schließlich umfassen Strukturelemente auch einen oder mehrere Iliakalgefäß-Grafts. Als Iliakalgefäß-Graft wird im Rahmen der Erfindung ein, in der Regel beim Einführen in den Körper separater, Graft bezeichnet, der bei der Implantation (im Körper des Patienten) an eine an dem Gefäßimplantat vorgesehene Bifurkation angeschlossen werden kann. Der Iliakalgefäß-Graft weist dabei ebenfalls einen tubulären Mantelkörper auf und vorzugsweise auch einen diesen umgebende Stentstruktur. Ein solcher Iliakalgefäß-Graft kann über die native Bifurkation in die Iliakalgefäße hineinragen und insbesondere dann eingesetzt werden, wenn auch ein Teil der Iliakalgefäße überbrückt werden soll. Ein erfindungsgemäßer Iliakalgefäß-Graft ist vorzugsweise mindestens 3 cm, mindestens 5, mindestens 7 cm oder mindestens 10 cm lang sein. Ein Iliakalgefäß-Graft kann wiederum weitere Strukturelemente wie Durchmesseränderungen, Aussparungen, Bifurkationen, Abzweigungen und lokale Verstärkungen beinhalten.

Insbesondere eine Kombination von lokaler Verstärkung und einem weiteren Strukturelement kann besonders vorteilhaft sein. Beispielsweise kann ein Modul eine Bifurkation und/oder Abzweigung mit mindestens einer lokalen Verstärkung aufweisen. Die lokale Verstärkung kann insbesondere am Übergang von tubulärem Mantelkörper in zwei tubuläre Mantelkörper beziehungsweise am Übergang vom Hauptmantelkörper zum Seitmantelkörper vorteilhaft sein, um die Dichtigkeit des Gefäßimplantats und die benötigte Festigkeit der Textilstruktur an der Übergangsstelle zu gewährleisten. Bei einer Fenestrierung ist insbesondere mindestens eine lokale Verstärkung in dem in Strömungsrichtung auf- und/oder abwärts gelegenen Bereich der Fenestrierung vorteilhaft, um die Last auf mindestens zwei Fäden zu verteilen und dadurch die Widerstandsfähigkeit bei der Implantation und/oder die Dauerfestigkeit der Fenestrierung zu gewährleisten. Dies kann durch eine Änderung der Bindung erzielt werden, beispielsweise durch den Einsatz einer Köperbindung an dem in Strömungsrichtung auf- oder abwärts gelegenen Bereich der Fenestrierung, besonders bevorzugt in beiden Bereichen. Alternativ oder zusätzlich kann der Einsatz der Köperbindung über mehrere Maschenreihen hinweg erfolgen und/oder bei der Abbindung der Ränder der Fenestrierungen im Gewirk des Mantelkörpers.

Im Falle einer manuellen Eingabe kann der Anwender ein oder mehrere Strukturelemente auswählen und optional in dem 3D-Modell, der technischen Zeichnung, einer 2D-Pixeldatei (z.B. einer Designdatei und/oder einer JC-Datei) oder der KMO Datei die gewünschte Position, Geometrie und/oder weitere Parameter zu dem jeweiligen Strukturelement angeben und/oder die jeweiligen Parameter mittels eines jeweiligen Computerprogramms bestimmen lassen. Das jeweilige Computerprogramm weist hierfür bevorzugt eine Abfrage in Form einer Eingabemöglichkeit auf. Mittels der Eingabemöglichkeit kann der Anwender beispielsweise ein oder mehrere Strukturelemente auswählen, beispielsweise in Form einer Dropdown-Liste, sowie optional je ausgewähltem Strukturelement jeweilige Parameter durch ein jeweiliges Computerprogramm bestimmen lassen und/oder manuell eingeben, bevorzugt mittels einer jeweiligen standardisierten Eingabemaske. Alternativ kann die Auswahl von Art und Position des einen oder der mehreren Strukturelemente mittels einer Drag-and-Drop Funktion im visualisierten 3D-Modell erfolgen. Dies erlaubt eine effiziente Abfrage jeweiliger Parameter in einem standardisierten Format. Ferner bietet die Eingabemöglichkeit bevorzugt auch die Möglichkeit kein Strukturelement einzugeben beziehungsweise auszuwählen. Im Anschluss kann die Herstellung des Gefäßimplantats erfolgen, beispielsweise nach einer finalen Bestätigung durch den Anwender.

Optional weist das jeweilige Computerprogramm eine Funktion auf, mittels derer die Notwendigkeit eines oder mehrerer Strukturelemente automatisch erkannt werden kann sowie jeweilige Strukturelemente ausgewählt und jeweilige Parameter optimal bestimmt und in das Modell integriert werden können. Eine solche Automatisierung reduziert mögliche Fehlerquellen und erlaubt eine zeit- und kosteneffiziente Herstellung eines patientenspezifischen Gefäßimplantats. Das Erkennen kann beispielsweise durch Abfrage von Grenzwerten und/oder in einer Datenbank gespeicherten Daten, beispielsweise Parametern zu jeweiligen Gefäßen, und Abgleich mit den entsprechenden jeweilig erhaltenen Gefäßparameter erfolgen. So kann beispielsweise ein jeweiliges Computerprogramm die Notwendigkeit eines Strukturelementes erkennen, wenn ein bestimmter Mindestabstand zwischen dem zu ersetzenden Gefäßabschnitt und einem abzweigenden Gefäß unterschritten wird und/oder der zu überbrückende beziehungsweise zu ersetzende Gefäßabschnitt eine Verzweigung aufweist. Wenn die Abfrage eines oder mehrerer Strukturelemente und die Bestimmung der jeweiligen Parameter automatisch erfolgen, so weist das jeweilige Computerprogramm bevorzugt eine Abfrage bezüglich der Zustimmung des Anwenders zu den vorgeschlagenen Strukturelementen und/oder dazu bestimmten Parametern auf sowie optional eine Korrekturmöglichkeit durch den Anwender. Dies kann beispielsweise nach dem Erkennen der Notwendigkeit mindestens eines Strukturelementes und/oder nach der Bestimmung der jeweiligen Parameter erfolgen. Wird keine Notwendigkeit eines oder mehrerer Strukturelemente erkannt, so kann unmittelbar nach einer möglichen Korrektur, insbesondere aber nach einer finalen Bestätigung durch den Anwender, die Herstellung des Gefäßimplantats anhand des erstellten Modells erfolgen.

Nach der Abfrage eines oder mehrerer Strukturelemente werden jeweilige bestimmte Parameter in das 3D-Modell, die technische Zeichnung und/oder die 2D-Pixeldatei, insbesondere in die farbcodierte 2D-Pixeldatei bevorzugt automatisch mittels eines jeweiligen Computerprogramms integriert und das resultierende Modell optional visualisiert. Bevorzugt kann der Anwender daraufhin, wenn nötig, eine Korrektur vornehmen und/oder die integrierten Strukturelemente mit den jeweiligen Parametern nach einer optischen Kontrolle bestätigen. Ferner erfolgt im Anschluss ein Abspeichern des jeweiligen Modells in einem von einer jeweiligen Maschine zur Herstellung des Gefäßimplantats, insbesondere einer Wirkmaschine, lesbaren Format, insbesondere in Form einer KMO Datei.

Durch die genannten Schritte des erfindungsgemäßen Verfahrens kann die Automatisierbarkeit, insbesondere eine Verringerung möglicher Fehlerquellen, verbesserte Qualitätskonstanz der herzustellenden Gefäßimplantate sowie Zeit- und Kosteneffizienz, bei gleichzeitigem Erhalt der vollen Kontrolle durch den jeweiligen Anwender verbessert werden.

Erfindungsgemäß ist das Gefäßimplantat (z.B. das endovaskuläre Gefäßimplantat) textil und/oder textilbasiert. Zur Erstellung eines Textils können dem Fachmann bekannte Techniken und Materialen verwendet werden, wobei das Gefäßimplantat bevorzugt aus biokompatiblen und/oder hämokompatiblen Materialien hergestellt ist. Beispiele hierfür sind Polytetrafluorethylen (PTFE), Polyethylenterephthalat (PET), Polyvinylidenfluorid (PVDF), Polyurethan (PU), Polylactid (PLA), Polycaprolacton (PCL), Ultra-High-Molecular-Weight Polyethylen (UHMWPE) und/oder Flüssigkristallpolymer (FKP, engl. LCP, liquid crystal polymer).

Das erfindungsgemäße Gefäßimplantat ist ein Gewebe und/oder eine Maschenware, bevorzugt eine Maschenware. Dabei wird unter einem "Gewebe" vorzugsweise Webware verstanden, die durch das rechtwinklige Verkreuzen von mindestens zwei Fadensystemen hergestellt wird. Der Begriff "Maschenware" bezeichnet hingegen Strick- und Wirkware, die einen oder mehrere verschlungene Fäden aufweist.

Für die Herstellung des Gefäßimplantats können beispielsweise Monofilament- und/oder Multifilamentgarne als Fäden verwendet werden. Die Filamentanzahl beträgt mindestens 22f, bevorzugt mindestens 36f, besonders bevorzugt mindestens 100f. In Bezug auf den Gesamttiter des Fadens können die Fäden beispielsweise gemäß EN ISO 2060:1995, bevorzugt nach der unter Punkt 4 genannten Variante 1 oder für Monofilamente beispielsweise gemäß EN 13392:2001 eine Feinheit von 1 dtex bis einschließlich 80 dtex, vorzugsweise bis einschließlich 60 dtex, besonders bevorzugt bis einschließlich 50 dtex aufweisen. Das Gefäßimplantat kann zum Beispiel nach seiner Herstellung eine Materialschichtdicke von 0,01 mm bis 2 mm, vorzugsweise von 0,1 mm bis 0,5 mm aufweisen, vorzugsweise optisch gemessen am Produkt und/oder bevorzugt mittels eines Dickenmessgeräts.

Das erfindungsgemäße Gefäßimplantat ist bevorzugt mittels Jacquard-Technik hergestellt, besonders bevorzugt mittels der Jacquard-Doppelrascheltechnik. Die Jacquard-Doppelrascheltechnik erlaubt eine besonders flexible Anpassung des Gefäßimplantats an die jeweilige Patientenanatomie.

Das erfindungsgemäße Verfahren umfasst ferner bevorzugt das Bestimmen von Positionen von Randabbindungen. Da die Gewirkherstellung erfindungsgemäß bevorzugt in zwei Flächen erfolgt, bezeichnen Randabbindungen Bereiche, in welchen jeweilige zwei Flächen miteinander verbunden werden, insbesondere parallel zur Produktionsrichtung der Gewirkherstellung. Fäden der Randabbindung bilden somit in jeder der jeweiligen Fläche Maschen aus, wodurch eine tubuläre Struktur ausgebildet werden kann. Der jeweilige mindestens eine tubuläre Hauptmantelkörper des mindestens einen Moduls des Gefäßimplantats kann somit mindestens zwei Randabbindungen aufweisen, mittels welcher die tubuläre Struktur des jeweiligen Moduls ausgebildet werden kann.

Zur Herstellung des Gefäßimplantats wird bevorzugt eine Doppelraschelmaschine mit Piezo-Jacquard-Technik verwendet. Ein von dieser Maschine lesbares Format stellt die KMO-Datei dar. Daher werden die Informationen des erstellten computergestützten Modells des Gefäßimplantats besonders bevorzugt als KMO-Datei, die, sofern zutreffend, in das Modell integrierte Parameter von mindestens einem Strukturelement aufweist, bevorzugt elektronisch an eine jeweilige Maschine übergeben beispielsweise mittels einer USB Schnittstelle. Nach Erhalt der zur Herstellung eines patientenspezifischen Gefäßimplantats benötigten Informationen in Form einer KMO-Datei oder einem anderen von der Maschine lesbaren Format wird das, z.B. endovaskuläre, Gefäßimplantat hergestellt.

Das erfindungsgemäße Gefäßimplantat weist bevorzugt eine Dichte in Produktionsrichtung angeordneter Maschen beziehungsweise eine Maschenreihendichte von mehr als 20 Maschen je einer Längeneinheit von 1 cm, bevorzugt von mehr als 25 Maschen je einer Längeneinheit von 1 cm, besonders bevorzugt von mehr als 30 Maschen je einer Längeneinheit von 1 cm gemessen am finalen Produkt auf. Die Maschen bilden dabei Fadenzwischenräume im Textil und bilden somit Poren des Gefäßimplantats aus. Die Größe und die Öffnungsfläche der Poren sind daher gut einstellbar, beispielsweise eine Porengröße von 1 µm bis 1000 µm, stärker bevorzugt von 10 µm bis 300 µm, und/oder eine Öffnungsfläche der Poren von 1 bis 1.000.000 µm², vorzugsweise von 100 bis 90.000 µm². Die Poren des Gefäßimplantats können dabei gleich und/oder verschieden groß ausgebildet sein und beispielsweise auch in Bezug auf ihre Größe entlang des mindestens einen tubulären Mantelkörpers des Gefäßimplantats variieren. So kann beispielsweise eine lagefeste Positionierung des Gefäßimplantats im Gefäß durch Einwachsen von nativem Gewebe in die Poren unterstützt werden.

Das Gefäßimplantat kann flüssigkeitsdurchlässig ausgestaltet sein. In diesem Fall bildet das Gefäßimplantat vorzugsweise ein selbstabdichtendes System, wobei das in dem Gefäß transportierte und/oder gespeicherte Fluid das Gefäßimplantat nach dessen Implantation abdichtet, beispielsweise durch Koagulation von in einer Flüssigkeit enthaltener Bestandteile in und/oder an dem Gefäßimplantat. Zu diesem Zweck kann beispielsweise eine Porengröße so gewählt sein, dass sie bei Implantierung des Gefäßimplantats in einem Blutgefäß beispielsweise durch eine Ablagerung von Fibrin im Rahmen der Blutgerinnung am Gefäßimplantat abgedichtet werden kann. Das Gefäßimplantat kann alternativ auch flüssigkeitsundurchlässig ausgestaltet sein, etwa mittels einer Beschichtung mit, beispielsweise biokompatiblem, Silikon, Polyurethan, Kollagen und/oder Gelatine.

In weiterer vorteilhafter Ausgestaltung des erfindungsgemäßen Verfahrens wird das Gefäßimplantat (z.B. das endovaskuläre Gefäßimplantat) einstückig gewirkt. In anderen Worten ist das erfindungsgemäße Verfahren zur Herstellung eines Gefäßimplantats (z.B. eines endovaskulären Gefäßimplantats) bevorzugt ferner dadurch charakterisiert, dass es ein einstückiges Wirken des Gefäßimplantats umfassend mindestens ein Modul mit jeweils mindestens einem tubulären Mantelkörper und jeweiligen ein oder mehreren Strukturelementen aufweist. So sind vorzugsweise die zwei tubulären Mantelkörper, in die sich das Gefäßimplantat an der Bifurkation verzweigt, einstückig mit dem vorhergehenden tubulären Abschnitt ausgebildet. Die zwei tubulären Mantelkörper der Bifurkation können hierbei mittels eines durchgängigen Gewirks (ohne Trennfäden) mit dem restlichen tubulären Mantelkörper des jeweiligen Moduls verbunden sein, es können jedoch bei Bedarf auch Trennfäden verwendet werden. Bei einer Abzweigung sind vorzugsweise der Haupt- und der Seitenkörper einstückig ausgebildet. Der Haupt- und Seitenkörper können hierbei mittels eines durchgängigen Gewirks (ohne Trennfäden) miteinander verbunden sein, es können jedoch bei Bedarf auch Trennfäden verwendet werden. Ebenso können die eine oder mehrere Verstärkungen, insbesondere bei Ausbildung als Köperbindung, einstückig mit dem Mantelkörper sein, insbesondere einstückig gewirkt.

Das einstückige Wirken des Gefäßimplantats umfassend das mindestens ein Modul mit jeweils mindestens einem tubulären Mantelkörper und jeweiligen ein oder mehreren Strukturelementen kann insbesondere durch die Jacquard-Technik erfolgen und bietet mehrere Vorteile. Ein Vorteil ist, dass das Gefäßimplantat keine herstellungsbedingten Schwachstellen aufweist, wie es beispielsweise beim Vernähen von Materialrändern der Fall sein kann. Ein weiterer Vorteil ist, dass weitere Arbeitsschritte bei der Herstellung des Gefäßimplantats entfallen, wie beispielsweise bei herkömmlichen Verfahren die Einbringung von Fenestrierungen und/oder Scallops mittels Ausstanzen und/oder Schneiden beispielsweise mittels Schneidewerkzeug und/oder Laser.

In weiterer vorteilhafter Ausgestaltung des erfindungsgemäßen Verfahrens weist dieses im Falle einer positiven Abfrage mindestens eines Strukturelements je nach dem mindestens einen ausgewählten Strukturelement, das in das herzustellende Gefäßimplantat integriert werden soll, weitere Schritte auf, die im Folgenden näher erläutert werden.

Soll das herzustellende Gefäßimplantat eine oder mehrere Bifurkationen und/oder eine oder mehrere Abzweigungen aufweisen, so umfasst das erfindungsgemäße Verfahren vorzugsweise ferner die Schritte: vorzugsweise Bestimmen von Geometrie und Bindung am Übergang vom Hauptmantelkörper zu der mindestens einen Bifurkation und/oder mindestens einen Abzweigung, Bestimmen von Positionen von Trennfäden; Einbringen von Trennfäden bei der Herstellung des Gefäßimplantats (z.B. des endovaskulären Gefäßimplantats); und Trennen der Trennfäden des hergestellten Gefäßimplantats.

Als "Trennfäden" werden im Kontext der vorliegenden Erfindung einzelne Maschenstäbchen bezeichnet, welche nicht durch eine Unterlegung mit anderen Maschenstäbchen verbunden sind, wobei eine Unterlegung die Verbindung zweier benachbarter Maschen, die aus demselben Faden gebildet werden, bezeichnet. Als Maschenstäbchen werden in Wirkrichtung übereinander angeordnete Maschen bezeichnet. Durch die Verwendung von Trennfäden ergeben sich in der Textilstruktur Trennlinien, welche ein definiertes Schneiden des Textils nach den jeweiligen Vorgaben zulassen. Aufgrund der Ausgestaltung der Gewirkstruktur bei der Nutzung von Trennfäden kann so das Risiko eines Aufribbelns beziehungsweise Auflösens der Textilstruktur verringert werden und durch die Wahl der Bindung eine Laufmaschenbildung ausgeschlossen werden. Ferner ist die Verwendung von Trennfäden in Kombination mit einer Änderung der Bindung, beispielsweise zur Einbringung von Aussparungen, lokalen Verstärkungen, Bifurkationen, Durchmesseränderungen und/oder Abzweigungen in ein Gefäßimplantat, besonders vorteilhaft, da sie eine einstückige Herstellung eines Gefäßimplantats (z.B. eines endovaskulären Gefäßimplantats) erlaubt, das mindestens ein Strukturelement ausgewählt aus der Gruppe bestehend aus Durchmesseränderung, Bifurkation, Abzweigung und Scallop aufweist. Wird das Gefäßimplantat mit mindestens einem Trennfaden gewirkt, so können die an den mindestens einen Trennfaden angrenzenden Bereiche des Gewirks bevorzugt mit mindestens einer lokalen Verstärkung ausgebildet werden, beispielsweise durch eine jeweilige Änderung der Fadenbindung. Dies ist insofern vorteilhaft, als dass eine Kombination von Trennfäden und lokaler Verstärkung das Risiko eines Auflösens der Struktur des Gewirks nach Trennung der Trennfäden minimiert. Durch die lokale Bindungsänderung kann eine vordere Textilfläche mit einer hinteren Textilfläche zur Herstellung eines Tubus verbunden werden. Die lokale Bindungsänderung kann sich von den erwähnten Randabbindungen unterscheiden.

Soll das herzustellende Gefäßimplantat mindestens eine Bifurkation und/oder Abzweigung und/oder mindestens eine Durchmesseränderung aufweisen, so umfasst das erfindungsgemäße Verfahren das Bestimmen von Positionen der in das Gefäßimplantat einzubringenden Trennfäden. Dies geschieht bevorzugt automatisch durch ein jeweiliges Computerprogramm, besonders bevorzugt im Rahmen des Bestimmens von jeweiligen Parametern der Bifurkation und/oder Abzweigung.

Ist mindestens eine Bifurkation als Strukturelement ausgewählt, bestimmt und/oder eingegeben worden, so kann das entsprechende Gefäßimplantat durch Änderung der Bindung und/oder unter Verwendung von Trennfäden einstückig gewirkt werden. Hierfür werden anhand der erhaltenen Gefäßparameter patientenspezifisch Position und Ausdehnung der Bifurkation bestimmt und bei Verwendung von Trennfäden diese so bestimmt, dass der tubuläre Mantelkörper des Gefäßimplantats in Wirkrichtung in zwei tubuläre Mantelkörper von, bei zweidimensionaler Betrachtung des Gewirks, z.B. vergleichbarer Breite beziehungsweise bei dreidimensionaler Betrachtung von z.B. vergleichbarem Durchmesser wie definiert aufgeteilt wird. Als Breite des Gewirks wird im Kontext der vorliegenden Erfindung die Ausdehnung orthogonal zur Haupterstreckungsrichtung des Gewirks bezeichnet. Zur Ausbildung einer Bifurkation kann beispielsweise die verwendete Fadenanzahl, gleichmäßig oder ungleichmäßig, auf jeweilige durch eine Bifurkation resultierende Bifurkationsmantelkörper aufgeteilt werden. Optional oder alternativ können zur Ausbildung einer Bifurkation Fäden entfernt oder hinzugenommen werden. Bei einer einstückigen Ausbildung der Bifurkationsmantelkörper wird vorzugsweise die Bindung lokal geändert. Der tubuläre Hauptmantelkörper des Gefäßimplantats kann zwei Randabbindungen aufweisen, mittels welcher die tubuläre Struktur des Gefäßimplantats ausgebildet werden kann, und die durch eine Bifurkation resultierenden Bifurkationsmantelkörper können jeweils zwei Randabbindungen aufweisen, mittels welchen die tubuläre Struktur des jeweiligen Bifurkationsmantelkörpers ausgebildet werden kann. Mindestens eine der zwei Randabbindungen des Hauptmantelkörpers kann in eine Randabbindung eines der Bifurkationsmantelkörper übergehen, insbesondere unterbrechungslos. Insbesondere können die beiden Randabbindungen des Hauptmantelkörpers in eine jeweilige äußere Randabbindung eines der beiden Bifurkationsmantelkörper übergehen, insbesondere unterbrechungslos.

Ist mindestens eine Abzweigung als Strukturelement ausgewählt, bestimmt und/oder eingegeben, so wird das entsprechende Gefäßimplantat wie im Fall einer Bifurkation vorzugsweise einstückig gewirkt. Hierfür kann ein einheitlicher tubulärer Mantelkörper des Gefäßimplantats im Bereich der Abzweigung in einen weiteren tubulären Mantelkörper (je nach Verfahrensführung in Wirkrichtung vor oder nach der Abzweigung) und einen tubulären Seitmantelkörper unterteilt werden. Zu diesem Zweck ist im Bereich der Abzweigung vorzugsweise eine Bindungsänderung vorgesehen. Der einheitliche tubuläre Mantelkörper, der weitere tubuläre Mantelkörper und der Seitmantelkörper werden hierbei vorzugsweise in der gleichen Richtung gewirkt. In anderen Worten beschrieben, erstrecken sich der weitere Mantelkörper und der Seitmantelkörper beim Wirken vorzugsweise parallel zum einheitlichen Mantelkörper der vor bzw. nach der Aufteilung gewirkt ist. Der weitere tubuläre Mantelkörper kann bei zweidimensionaler Betrachtung des Gewirks eine geringere Breite beziehungsweise bei dreidimensionaler Betrachtung einen geringeren Durchmesser als der tubuläre Mantelkörper aufweisen.

Alternativ oder zusätzlich kann der Seitmantelkörper durch hinzugenommene Fäden einstückig hergestellt werden. Hierunter werden insbesondere Fäden verstanden, die jedenfalls in den Bereichen unmittelbar vor und/oder unmittelbar nach der jeweiligen Abzweigung nicht zum Wirken des tubulären Hauptmantelkörpers verwendet werden. Vorzugsweise sind beim Wirken des tubulären Hauptmantelkörpers (in Wirkrichtung vor und/oder nach dem Bereich der Abzweigung) mehrere Trennfäden vorgesehen (die z.B. parallel zur Haupterstreckungsrichtung des Hauptmantelkörpers gewirkt werden können). Bevorzugt werden die als Trennfäden auszubildenden Fäden parallel zum Hauptmantelkörper mit derselben Bindung wie der jeweilige Hauptmantelkörper gewirkt und unmittelbar benachbart zur jeweiligen Abzweigung als Trennfäden ausgeführt. Dies ermöglicht ein qualitativ besonders hochwertiges Warenbild und eine robuste Prozessführung. Unter Verwendung dieser Trennfäden wird dann an einer gewünschten Stelle ein Seitmantelkörper gewirkt, der beim Wirken mit dem Hauptmantelkörper verbunden wird. Somit kann aus den (vorzugsweise mehreren) Trennfäden ein Seitmantelkörper gewirkt werden, der an einer gewünschten Position in Längsrichtung des Hauptmantelkörpers an diesem entspringt. Der Seitmantelkörper kann hierbei bereits beim Wirken in einem gewünschten Winkel zum Hauptmantelkörper (z.B. 0° bis 75°, 5° bis 75° oder 10° bis 75°, vorzugsweise 0° bis 45°, 5° bis 45° oder 10° bis 45°) einstückig hergestellt werden. Der Durchmesser des Hauptmantelkörpers kann über den Bereich der Abzweigung hinweg im Wesentlichen konstant bleiben. Vorzugsweise erfolgt die Herstellung des Seitmantelkörpers dabei ohne eine Reduktion und/oder Aufteilung der für den Hauptmantelkörper verwendeten Fadenzahl. Optional kann derselbe Trennfaden oder können dieselben Trennfäden an verschiedenen Stellen entlang eines jeweiligen Moduls und/oder des Implantats zur Ausbildung verschiedener Strukturelemente verwendet werden, etwa zur Ausbildung mehrerer Abzweigungen oder zur Ausbildung einer Abzweigung und einer Bifurkation.

Unabhängig von der Art der Herstellung kann der Seitmantelkörper eine geringere Breite beziehungsweise einen geringeren Durchmesser als der weitere tubuläre Mantelkörper aufweisen.

Die zuvor bestimmte Position der Trennfäden wird in das computergestützte Model integriert, bevorzugt insbesondere in die farbcodierte 2D-Pixeldatei. Entsprechend der in dem Model integrierten Informationen, insbesondere bezüglich Anordnung am tubulären Mantelkörper und Ausdehnung, werden die Trennfäden bei der Herstellung des Gefäßimplantats (z.B. des endovaskulären Gefäßimplantats) in selbiges eingebracht. Das Gefäßimplantat wird somit entsprechend des computergestützten Models mit Trennfäden gewirkt.

Nach der Herstellung des Gefäßimplantats werden die in dem Gefäßimplantat enthaltenen Trennfäden vorzugsweise getrennt. Das Trennen der Trennfäden des hergestellten Gefäßimplantats kann über verschiedene dem Fachmann bekannte Methoden erfolgen, beispielsweise durch Schneiden. Bevorzugt erfolgt das Trennen der Trennfäden automatisch, es kann jedoch auch manuell erfolgen.

Bei dem erfindungsgemäßen Verfahren können die eine oder mehreren Abzweigungen und/oder Bifurkationen parallel zur Haupterstreckungsrichtung des jeweiligen Moduls und/oder Implantats gewirkt werden. Die Abzweigung oder Bifurkation kann durch eine nachfolgende Thermoformung und/oder Thermofixierung in einem Winkel der Haupterstreckungsrichtung angeordnet werden. Alternativ oder zusätzlich können die eine oder mehreren Abzweigungen und/oder Bifurkationen mit einem Winkel zur Haupterstreckungsrichtung des jeweiligen Moduls und/oder Implantats gewirkt werden, insbesondere durch einen Einsatz von Trennfäden.

Soll das herzustellende Gefäßimplantat eine oder mehrere Aussparungen aufweisen, so umfasst das erfindungsgemäße Verfahren vorzugsweise ferner die Schritte: Bestimmen von Positionen von mindestens einer lokalen Verstärkung an den Rändern der mindestens einen Aussparung und/oder Einbringen von Trennfäden. Wenn die mindestens eine Aussparung eine Fenestrierung sein soll, umfasst das Verfahren vorzugsweise das Einbringen von mindestens einer lokalen Verstärkung bei der Herstellung des Gefäßimplantats. Wenn die mindestens eine Aussparung ein Scallop sein soll, umfasst das Verfahren vorzugsweise das Bestimmen von Positionen von Trennfäden, das Einbringen von lokalen Verstärkungen und Einbringen von Trennfäden bei der Herstellung des Gefäßimplantats sowie das Trennen der Trennfäden des hergestellten Gefäßimplantats.

Eine Aussparung in einem erfindungsgemäßen Gefäßimplantat weist besonders bevorzugt mindestens eine lokale Verstärkung an den Rändern der mindestens einen Aussparung auf. So weist eine Fenestrierung bevorzugt eine lokale Verstärkung in dem in Strömungsrichtung auf- oder abwärts gelegenen Bereich der Fenestrierung auf, besonders bevorzugt in beiden Bereichen. Ein Scallop weist bevorzugt eine lokale Verstärkung in dem in Strömungsrichtung abwärts gelegenen Bereich des Scallops auf. Die entsprechenden Parameter, insbesondere die jeweilige Position der mindestens einen lokalen Verstärkung, werden bevorzugt automatisch bestimmt und in das computergestützte Modell des Gefäßimplantats integriert. Besonders bevorzugt wird eine lokale Verstärkung durch eine Änderung der Bindung ausgestaltet, insbesondere durch eine Köperbindung (insbesondere am oberen und/oder unteren Ende). Entsprechend der in dem Model integrierten Parameter wird das Gefäßimplantat somit entsprechend des Models einstückig mit lokalen Verstärkungen gewirkt.

Ist mindestens eine Aussparung ein Scallop, so kann das Gefäßimplantat unter Verwendung von Trennfäden einstückig gewirkt werden. Hierfür werden anhand der erhaltenen Gefäßparameter patientenspezifisch Position und Ausdehnung der Trennfäden so bestimmt, dass die den mindestens einen Scallop unmittelbar umlaufenden Maschen als Maschen jeweiliger Trennfäden ausgebildet werden. Auf diese Weise kann der Bereich des tubulären Mantelkörpers, der an der Stelle des Scallops gewirkt ist, nach der Herstellung des Gefäßimplantats herausgetrennt werden. Das Einbringen von lokalen Verstärkungen und das Einbringen von Trennfäden bei der Herstellung des Gefäßimplantats sowie dem anschließenden Trennen der Trennfäden kann dabei wie bereits beschrieben erfolgen.

Soll das herzustellende Gefäßimplantat eine oder mehrere Durchmesseränderungen aufweisen, so umfasst das erfindungsgemäße Verfahren vorzugsweise ferner die Schritte: Bestimmen von Parametern zur Fadenspannung, Fadenanzahl, Maschenreihendichte und/oder Maschengröße. Alternativ oder zusätzlich umfasst das Verfahren vorzugsweise die Schritte: Bestimmen von Positionen von Trennfäden, Einbringen von Trennfäden bei der Herstellung des Gefäßimplantats (z.B. des endovaskulären Gefäßimplantats), und Trennen der Trennfäden des hergestellten Gefäßimplantats.

Zusätzlich oder alternativ zu der bereits beschriebenen Option der Verwendung von Trennfäden kann eine Durchmesseränderung durch Änderung von Parametern zu Fadenspannung, Fadenanzahl, Maschenreihendichte und/oder Maschengröße ausgebildet werden. Beispielsweise kann eine Reduktion des Durchmessers des Gefäßimplantats durch eine Erhöhung der Fadenspannung, eine Reduktion der Fadenanzahl beispielsweise durch die Nutzung von Trennfäden, eine Erhöhung der Maschenreihendichte oder eine Reduktion der Maschengröße erzielt werden oder aus einer Kombination einer oder mehrerer der zuvor genannten Möglichkeiten. Ist hingegen eine Vergrößerung des Durchmessers nötig, so kann dies durch eine Reduktion der Fadenspannung, eine Erhöhung der Fadenanzahl, eine Reduktion der Maschenreihendichte und/oder eine Erhöhung der Maschengröße ausgestaltet werden.

Das erfindungsgemäße Gefäßimplantat kann mehrere Module umfassen, die einstückig gewirkt werden und über Trennfäden verbunden sind. Diese Module können nach dem Wirken durch Trennen der Trennfäden voneinander getrennt werden. So kann z.B. ein tubulärer Hauptmantelkörper (optional mit einer oder mehreren Abzweigungen, einer oder mehreren Fenestrierungen, einem oder mehreren Scallops und/oder einer oder mehreren Durchmesseränderungen) ein erstes Modul bilden und die Bifurkation als ein zweites Modul ausgebildet sein, das über Trennfäden mit dem ersten Modul verbunden ist. Ein weiteres Modul kann ein tubulärer Mantelkörper für einen in das rechte Iliakalgefäß einzusetzender Graft sein und/oder ein weiteres Modul kann ein tubulärer Mantelkörper für einen in das linke Iliakalgefäß einzusetzender Graft sein. Der bzw. die tubulären Mantelkörper für den oder die Iliakalgefäß-Grafts können über Trennfäden mit dem ersten Modul, insbesondere mit den Enden der Bifurkation, verbunden sein.

Alternativ kann der tubuläre Hauptmantelkörper (optional mit einer oder mehreren Abzweigungen, einer oder mehreren Fenestrierungen, einem oder mehreren Scallops und/oder einer oder mehreren Durchmesseränderungen) ein erstes Modul bilden, das unmittelbar eine Bifurkation aufweist (d.h. ohne, dass der tubuläre Hauptmantelkörper und die Bifurkation nach dem Wirken getrennt werden) und ein weiteres Modul durch einen tubulären Mantelkörper für einen in das rechte Iliakalgefäß einzusetzender Graft sein und/oder ein weiteres Modul durch einen tubulären Mantelkörper für einen in das linke Iliakalgefäß einzusetzender Graft gebildet sein.

Der bzw. die tubulären Mantelkörper für den oder die Iliakalgefäß-Grafts können über Trennfäden mit dem ersten Modul, insbesondere mit den Enden der Bifurkation, verbunden sein. Gemäß einer weiteren Alternative kann ein tubulärer Hauptmantelkörper (optional mit einer oder mehreren Abzweigungen, einer oder mehreren Fenestrierungen, einem oder mehreren Scallops und/oder einer oder mehreren Durchmesseränderungen) ein erstes Modul bilden und ein in eines der Iliakalgefäße einzusetzender Graft ein zweites Modul, das beim Wirken über Trennfäden mit dem ersten Modul verbunden ist. Die unterschiedlichen Module werden vorzugsweise im Körper überlappend expandiert, wodurch diese verbunden werden.

Soll das erfindungsgemäße Gefäßimplantat mindestens einen Iliakalgefäß-Graft aufweisen, wird dieser vorzugsweise integral mit dem restlichen Gefäßimplantat gewirkt und anschließend vom restlichen Gefäßimplantat getrennt, um separat in den Körper eingesetzt werden zu können. Dies ermöglicht eine kostengünstige Herstellung patientenindividualisierter Iliakalgefäß-Grafts. So kann insbesondere die Länge und/oder der Durchmesser des Grafts als patientenindividueller Parameter bestimmt werden. Je nach den individuellen anatomischen Erfordernissen des Patienten kann der Iliakalgefäß-Graft mit einem konstanten Durchmesser oder mit einer oder mehreren Durchmesseränderungen ausgebildet werden. Weiterhin kann ein Iliakalgefäß-Graft mindestens eine Aussparung und/oder mindestens eine Abzweigung aufweisen.

Um eine einfache Trennbarkeit des Iliakalgefäß-Grafts vom übrigen Gefäßimplantat zu erreichen, wird ein integral gewirkter Iliakalgefäß-Graft vorzugsweise über Trennfäden mit dem übrigen Graft verbunden. So kann insbesondere ein Ende des integral gewirkten Iliakalgefäß-Grafts über Trennfäden mit einem Ende eines integral mit dem übrigen Gefäßimplantat gewirkten Bifurkationsmantelkörpers verbunden sein. Die Trennfäden werden dann nach dem Wirken getrennt, um einen separat in den Körper des Patienten einführbaren Iliakalgefäß-Graft zu erhalten.

In weiterer vorteilhafter Ausgestaltung des erfindungsgemäßen Verfahrens weist dieses ferner die folgenden Schritte auf: Erhalten pre-operativer medizinischer Bilddaten eines Gefäßes; Visualisieren der erhaltenen Bilddaten; Identifizieren eines Gefäßabschnittes, für den ein Gefäßimplantat (z.B. ein endovaskuläres Gefäßimplantat) hergestellt werden soll; und Messen von Gefäßparametern. Auch wenn mit dem erfindungsgemäßen Verfahren standardisierte Gefäßimplantate (insbesondere standardisierte endovaskuläre Gefäßimplantate) hergestellt werden können, so wird es doch bevorzugt zur Herstellung individualisierter Gefäßimplantate verwendet. So kann der patientenspezifischen Anatomie in dem betroffenen Abschnitt besonders gut Rechnung getragen werden. Dies ist insbesondere im Falle von Verzweigungen des zu ersetzenden Gefäßabschnittes vorteilhaft, da Ausbildung und Lage der Verzweigungen stark variieren können. Bevorzugt wird das Gefäßimplantat daher anhand von Bilddaten von mindestens dem Gefäßabschnitt, für den ein Gefäßimplantat (z.B. ein endovaskuläres Gefäßimplantat) hergestellt werden soll, erzeugt. So können insbesondere Struktur und/oder Form des Gefäßimplantats wie beispielsweise Lage, Form und/oder Größe eines oder mehrerer Strukturelemente basierend auf solchen Bilddaten patientenindividualisiert ausgestaltet und/oder hergestellt werden.

Bevorzugt werden daher im Rahmen des erfindungsgemäßen Verfahrens preoperative medizinische Bilddaten mindestens des zu ersetzenden Gefäßabschnittes erhalten, bevorzugt mindestens des zu ersetzenden Gefäßabschnittes einschließlich unmittelbar angrenzender Gefäßabschnitte. Die Bilddaten werden bevorzugt mittels Computer- oder Magnetresonanztomographie, optional aber auch mittels Röntgen- oder Ultraschallvorrichtungen erhalten, vor allem wenn sie zur Aufnahme und/oder Erstellung eines 3D-Bilddatensatzes geeignet sind. Als Grundlage für die Herstellung eines individualisierten Gefäßimplantats weisen die Bilddaten vorzugsweise eine Schichtdicke von maximal 3 mm, bevorzugt von maximal 2 mm und besonders bevorzugt von maximal 1 mm auf. Die Daten werden vorzugsweise in Longitudinalrichtung des Körpers, d.h. von cranial (kopfwärts) nach caudal (steißwärts) oder in umgekehrter Richtung in einem Abstand von 0,05 mm bis 10 mm, bevorzugt in einem Abstand von 0,5 mm bis 3 mm aufgenommen. Zwei aufeinanderfolgende Schichten können eine Überlappung von maximal 50 %, bevorzugt von maximal 25 % aufweisen, wobei der Abstand zwischen den Schichten maximal so groß wie die entsprechende Schichtdicke ist. Um hochqualitative Informationen hinsichtlich der exakten Ausgestaltung der Gefäßwand zu gewährleisten, werden die gewonnenen und/oder erhaltenen Bilder bevorzugt auf eine dem Fachmann bekannte Art nachbearbeitet, beispielsweise mittels multiplanarer Rekonstruktion, um eine möglichst hohe räumliche Auflösung in alle Raumrichtungen bereitzustellen, bevor sie zur Herstellung eines individualisierten Gefäßimplantats herangezogen werden.

Die gewonnenen und/oder bereitgestellten Bilddaten können dabei zu einem oder mehreren Zeitpunkten erstellt worden sein und werden in einem bevorzugten erfindungsgemäßen Verfahren zur Herstellung eines computergestützten Modells des Gefäßimplantats herangezogen, um ein individualisiertes Gefäßimplantat auszugestalten.

Bevorzugt werden die erhaltenen pre-operativen medizinischen Bilddaten ferner visualisiert. Eine optische Darstellung der Daten erleichtert die Kontrolle der erhaltenen Datenqualität sowie der Vollständigkeit der erhaltenen Daten. Im Falle einer für die Erstellung eines computergestützten Modells nicht ausreichenden Datenqualität oder unvollständiger Daten kann der Anwender so beispielsweise alternativ oder zusätzlich durch die Ausgabe eines jeweiligen Hinweises gewarnt werden. Dadurch kann zeitnah ein erneutes Erhalten pre-operativer medizinischer Bilddaten des zu ersetzenden Gefäßabschnittes sowie der unmittelbar daran angrenzenden Gefäßabschnitte veranlasst werden, wodurch die Bereitstellung eines patientenspezifischen Gefäßimplantats besonders zeiteffizient erfolgen kann.

Das erfindungsgemäße Verfahren kann ferner das, bevorzugt automatische, Identifizieren eines Gefäßabschnittes, für den ein Gefäßimplantat (z.B. ein endovaskuläres Gefäßimplantat) erstellt werden soll, aufweisen. Der zu ersetzende Gefäßabschnitt wird anhand der erhaltenen Bilddaten bevorzugt mittels eines in einem computergestützten Programm implementierten Verfahrens identifiziert. Dies kann aber auch manuell durch eine entsprechende Markierung und/oder Eingabe des Anwenders erfolgen. Bevorzugt erfolgt die Identifizierung jedoch automatisch, beispielsweise durch eine automatische Segmentierung des zu behandelnden Gefäßes und/oder Abgleich von Grauwerten. Auch kann beispielsweise ein Abgleich mit Einträgen einer Datenbank, die Grenzwerte für jeweilige Gefäßparameter beinhaltet, erfolgen. Zudem können, wenn vorhanden, patientenspezifische Gefäßparameter zum Vergleich herangezogen werden, die der zu ersetzende Gefäßabschnitt aufgewiesen hat, bevor die Gefäßwandveränderung eintrat. Dies ermöglicht eine maximale patientenspezifische Anpassung des herzustellenden Gefäßimplantats.

Bevorzugt wird der zu ersetzende Gefäßabschnitt in der Visualisierung der Bilddaten optisch mit ausgegeben. Auf diese Weise kann der Anwender den automatisch identifizierten Gefäßabschnitt kontrollieren und gegebenenfalls korrigieren, beispielsweise verlängern, verkürzen, erweitern oder einschränken. Eine Abfrage, ob der Anwender zustimmt, mit dem erfindungsgemäßen Verfahren fortzufahren, kann vorgesehen und entsprechend in dem jeweiligen Computerprogramm implementiert sein.

Ist der zu ersetzende Gefäßabschnitt identifiziert, und bevorzugt auch durch den Anwender bestätigt worden, so erfolgt vorzugsweise das Messen von jeweiligen Gefäßparametern des zu ersetzenden Gefäßabschnittes. Hierfür kann beispielsweise zunächst eine Centerline des zu behandelnden Gefäßes bestimmt werden, die optional auch als Rotationsachse mindestens eines der tubulären Mantelkörper des herzustellenden Gefäßimplantats gemäß dessen Haupterstreckungsrichtung angesehen werden kann. Eine Centerline kann jedoch auch abweichend von einer Mittellinie des zu behandelnden Gefäßes bestimmt werden, beispielsweise um mechanische Eigenschaften von Geräten zu berücksichtigen, die für eine Implantation des Gefäßimplantats verwendet werden und/oder mechanische Eigenschaften des Gefäßimplantats selbst. Ferner können Gefäßparameter anhand der erhaltenen medizinischen Bilddaten und optional unter Zuhilfenahme der bestimmten Mittellinie des zu ersetzenden Gefäßes gemessen werden, wie beispielsweise Länge und Durchmesser des Gefäßes sowie Lage, Winkel und Durchmesser von Gefäßverzweigungen. Aus solchen Parametern können anschließend Parameter des Gefäßimplantats berechnet werden, etwa Länge und Durchmesser des einen oder der mehreren Mantelkörper und/oder die Lage und der Durchmesser von Aussparungen. Neben solchen bereits beschriebenen geometrischen Parametern können optional auch morphologische Parameter erfasst werden, wie beispielsweise das Vorhandensein von Thromben und/oder Kalzifizierung sowie der jeweiligen Lage, Geometrie und Ausdehnung.

Die gemessenen Gefäßparameter werden bevorzugt gespeichert und zur Erstellung des computergestützten Modells des Gefäßimplantats, wie bereits ausgeführt, bereitgestellt. Die gemessenen Gefäßparameter können insbesondere auch bei der Ermittlung von Strukturelementen wie lokalen Verstärkungen analysiert werden und entsprechend in das Model des Gefäßimplantats (z.B. des endovaskulären Gefäßimplantats) integriert werden.

Handelt es sich bei dem herzustellenden Gefäßimplantat um einen Stentgraft und/oder um ein Klappenimplantat, wie beispielsweise eine Herzklappe, so umfasst das erfindungsgemäße Verfahren in weiterer vorteilhafter Ausgestaltung vorzugsweise ferner das Aufbringen einer Stentstruktur auf das hergestellte textile und/oder textilbasierte Gefäßimplantat und/oder einer Klappe an dem hergestellten Gefäßimplantat. Der Begriff "Stentstruktur" oder auch "Stent" bezeichnet hierbei eine Struktur, die vorzugsweise eine Mehrzahl von Streben aufweist und tubulär und/oder tubusförmig ausgebildet ist. Eine Stentstruktur ist dabei ebenso wie eine Klappe bevorzugt aus einem biokompatiblen Material, insbesondere einem biokompatiblen polymerbasierten Material, kann alternativ jedoch beispielsweise auch aus Metall sein und/oder Metall umfassen. Insbesondere im Fall einer Stentstruktur ist diese bevorzugt patientenspezifisch ausgestaltet unter Berücksichtigung gemessener Gefäßparameter, insbesondere geometrischer sowie optional auch morphologischer Gefäßparameter. Hierbei kann beispielsweise auch eine Visualisierung insbesondere des erstellten 3D-Modells des zu wirkenden Gefäßimplantats, und damit des Grafts, für eine weitere Produktauslegung in Anschluss an die Textilherstellung, wie beispielsweise eine geeignete Positionierung einer Stentstruktur, vorteilhaft sein. Im visualisierten Modell kann die Positionierung einer Stentstruktur einfach vorgenommen werden und daraus beispielsweise eine technische Zeichnung für das Design und die Auslegung des Stents und für die nachfolgende Assemblierung von Graft und Stent erstellt werden. Auch eine Simulation der Implantation und/oder des Verhaltens des Gefäßimplantats unter mechanischer Belastung wie beispielsweise während oder nach einer Implantation kann für eine optimale Ausgestaltung einer Stentstruktur eines Stentgrafts vorteilhaft sein. Das Aufbringen einer Stentstruktur und/oder das Anbringen einer Klappe auf einen erfindungsgemäß hergestellten Graft kann auf verschiedene, dem Fachmann bekannte Arten erfolgen, beispielsweise mittels Nähen, Kleben, Verschweißen oder Verschmelzen.

In weiterer vorteilhafter Ausgestaltung des erfindungsgemäßen Verfahrens wird die Stentstruktur auf das hergestellte Gefäßimplantat gedruckt. In diesem Fall ist die Stentstruktur bevorzugt aus einem polymerbasierten, insbesondere biokompatiblen, Material und das Aufbringen der Stentstruktur auf das Gefäßimplantat, in diesem Fall einen sogenannten Graft, erfolgt mittels eines additiven Verfahrens, insbesondere mittels Fused Deposition Modeling. Im Rahmen der vorliegenden Erfindung beinhaltet der Begriff "additives Verfahren" dem Fachmann bekannte additive Fertigungsverfahren beziehungsweise Verfahren zur additiven und/oder generativen Fertigung, bei denen Elemente durch Auf- und/oder Aneinanderfügen von Material automatisiert hergestellt werden. Additive Verfahren umfassen beispielsweise 3D-Druck Verfahren sowie Schmelzschichtungsverfahren wie das Fused Deposition Modeling. Beim Fused Deposition Modeling wird das Material der Stentstruktur beispielsweise durch eine beheizte Düse verflüssigt und in Form von Filamenten in einer oder mehreren Schichten auf den Graft aufgebracht, vorzugsweise unmittelbar und/oder direkt auf den Graft. Das Material kann der Düse als Filament zugeführt werden.

Das Aufbringen der Stentstruktur auf den Graft erfolgt bevorzugt durch den Einsatz eines rotierbaren Halters, mittels dem der Graft gehalten und/oder gedreht werden kann, vorzugsweise um seine Längsachse. Bei einem solchen Halter kann es sich beispielsweise um einen rotierenden tubulären Kern und/oder um ein rotierendes tubuläres Gerüst handeln, auf dessen Außenfläche der bereitgestellte Graft aufliegt. In diesem Fall wird die mindestens eine Stentstruktur bevorzugt außen auf den Graft aufgebracht. Sofern die aufgebrachte Stentstruktur innerhalb des Grafts angeordnet werden soll, kann das erfindungsgemäße Verfahren einen weiteren, optionalen Schritt des Umstülpens des Stentgrafts umfassen. Der Graft des erfindungsgemäßen Stentgrafts kann daher so vorgesehen werden, dass er die Stentstruktur von innen oder außen ummantelt.

In weiterer vorteilhafter Ausgestaltung des erfindungsgemäßen Verfahrens weist dieses ferner Schritte zur Formgebung und/oder Fixierung des Gefäßimplantats (z.B. des endovaskulären Gefäßimplantats) mittels Hitzebehandlung und/oder Erhitzen des hergestellten Gefäßimplantats (z.B. des hergestellten endovaskulären Gefäßimplantats) auf. In anderen Worten weist das erfindungsgemäße Verfahren bevorzugt einen Schritt der Thermoformung und/oder Thermofixierung des, z.B. endovaskulären, Gefäßimplantats auf, insbesondere im Falle einer oder mehrerer Durchmesseränderungen entlang des tubulären Mantelkörpers. Im Kontext der vorliegenden Erfindung erfolgt eine Thermofixierung nach einer jeweiligen Textilherstellung. Ferner bezeichnet im Kontext der vorliegenden Erfindung der Begriff Thermoformen eine Formgebung des Textils durch eine Kombination einer jeweiligen Kerngeometrie des Halters und eines Wärmeeinflusses, insbesondere eines Wärmeeinflusses von mindestens 120 °C oder mindestens 150 °C. Ein Schritt der Thermoformung und/oder Thermofixierung ist besonders vorteilhaft, um die Form des Implantats nach der Herstellung zu erhalten und/oder zu fixieren. Dabei kann die Form des gewebten oder gewirkten Implantats insbesondere auch weiter an die Form des zu behandelnden Gefäßes angepasst werden. So kann beispielsweise die Form der einen oder mehreren Aussparungen angepasst, insbesondere aufgeweitet werden. Alternativ oder zusätzlich kann der Winkel von Bifurkationen und/oder Abzweigungen relativ zur Haupterstreckungsrichtung des Implantats angepasst und fixiert werden. Alternativ oder zusätzlich kann insbesondere der jeweilige Durchmesser des einen oder der mehreren Mantelkörper angepasst werden, insbesondere aufgeweitet werden. Zudem kann die Form des Gefäßimplantats am Übergang des Hauptmantelkörpers zu einem oder mehreren Bifurkations- und/oder Seitmantelkörpern durch eine Thermoformung und/oder Thermofixierung in einer gewünschten Form (z.B. in einer gerundeten und/oder stufenlosen Form) fixiert werden. Somit kann durch einen Schritt zur Formgebung und/oder Fixierung des, z.B. endovaskulären, Gefäßimplantats allein und/oder in Kombination mit Einstellmöglichkeiten bezüglich der Gewirkherstellung, wie Bindung und Einbringung mindestens eines Strukturelements wie vorstehend beschrieben, eine optimale, patientenspezifische Ausbildung des Gefäßimplantats erzielt werden.

Für eine Formgebung und/oder Fixierung wird das hergestellte Gefäßimplantat bevorzugt von einem Halter gehalten, der vorzugsweise rotierbar und/oder drehbar ist, vorzugsweise um seine Längsachse. Der Halter hat dabei bevorzugt mindestens einen Durchmesser, der dem des zu ersetzenden Gefäßabschnittes entspricht. Zur Herstellung von Durchmesseränderungen mittels Thermoformung und/oder Thermofixierung kann der Halter auch Bereiche mit verschiedenen Durchmessern (z.B. Durchmessersprünge) aufweisen.

Besonders bevorzugt wird ein Halter verwendet, der mindestens einen Kern mit einer jeweiligen Kerngeometrie gemäß dem erstellten computergestützten Modell des hergestellten Gefäßimplantats aufweist. Ein Halter kann somit einen modularen Aufbau aufweisen (z.B. durch die Verbindung verschiedener Halterabschnitte mit unterschiedlichen Durchmessern und/oder durch die Verbindung eines Hauptabschnitts zur Fixierung des Hauptmantelkörpers mit Bifurkations- und/oder Abzweigungsabschnitten zur Fixierung von Bifurkationen bzw. von Abzweigungen des hergestellten Grafts). Ein jeweiliger Halter kann beispielsweis mittels additiver Fertigung oder auf Basis spanender Verfahren anhand des erstellten computergestützten Modells des hergestellten Gefäßimplantats erstellt werden. Dies hat den Vorteil, dass schnell und kostensparend Halter mit einer Kerngeometrie erzeugt und verwendet werden können, die eine optimale Formgebung des hergestellten Gefäßimplantats an die jeweilige Patientenanatomie und/oder die Fixierung eben dieser Form zur Sicherstellung der Funktion des Gefäßimplantats ermöglichen. Bei einem solchen Halter kann es sich beispielsweise um einen rotierenden tubulären Kern und/oder ein rotierendes tubuläres Gerüst handeln, auf dessen Außenfläche das Gefäßimplantat aufliegt. Der Halter kann als Vollkörper ausgestaltet sein. Bevorzugt handelt es sich bei dem Halter jedoch um einen, vorzugsweise dünnwandigen, hohlen Kern. Der Halter hat - unabhängig davon, ob er als Vollkörper oder mit einem hohlen Kern ausgebildet ist - optional Verzweigungen gemäß der im Graft eingebrachten Verzweigungen. Ein dünnwandiger, hohler Kern ist besonders vorteilhaft, da er eine homogene Wärmeleitung gewährleisten kann und somit eine gleichmäßige Thermoformung und/oder Thermofixierung des aufgelegten Gefäßimplantats sicherstellt. Bevorzugt ist der Halter aus Edelstahl und/oder weist Edelstahl auf. Der Halter kann auch aus verschiedenen Materialien bestehen und/oder diese aufweisen, beispielsweise kann der Halter eine AluminiumLegierung aufweisen, die von einer PTFE-Folie umgeben sein kann. Dadurch können Unebenheiten der Oberfläche reduziert werden und es kann eine glatte Oberfläche des Halters sichergestellt werden.

Das Gefäßimplantat kann somit auf einen Halter aufgelegt und/oder mit einer definierten Vordehnung aufgespannt werden, wobei die definierte Vordehnung beispielsweise 20 % betragen kann, bevorzugt 30 %. Besonders bevorzugt wird eine definierte Vordehnung unter Berücksichtigung jeweiliger Textilparameter und/oder mittels jeweiliger Kerngeometrie des Halters lokal angepasst. Der Halter wird daraufhin mit dem darauf aufgelegten Gefäßimplantat entsprechend materialspezifischer vordefinierter Parameter und/oder halterspezifischer vordefinierter Parameter, etwa entsprechend des jeweiligen Materials, der jeweiligen Geometrie und/oder der jeweiligen Ausgestaltung des mindestens einen Kerns beispielsweise als Hohl- oder Vollkern, insbesondere Temperatur und Dauer, erhitzt, bevorzugt in einem Ofen, besonders bevorzugt in einem Trockenluft-Ofen. Anschließend wird der Halter zusammen mit dem darauf aufgelegten Gefäßimplantat beispielsweise aus dem Ofen herausgenommen, bevorzugt bei Raumtemperatur abgekühlt und das Gefäßimplantat abgenommen beispielsweise durch Abziehen von dem jeweiligen Halter. Durch die kontrollierte Wärmebehandlung des Gefäßimplantats im Rahmen der Thermoformung und/oder Thermofixierung kann eine kontrollierte Schrumpfung des Gefäßimplantats in seiner Haupterstreckungsrichtung beziehungsweise der Wirkrichtung des textilen und/oder textilbasierten Gefäßimplantats erfolgen und/oder fixiert werden. Durch eine solche Schrumpfung werden insbesondere Aussparungen in ihre jeweilige vorgesehen Geometrie gebracht, bei hergestellten Fenestrierungen beispielsweise von gewirkten Schlitzen in jeweilige ovale oder kreisförmige Öffnungen. Besonders vorteilhaft kann ein Gefäßimplantat, das mit einem Hauptmantelkörper gewirkt ist, der eine gleichbleibende oder im Wesentlichen gleichbleibende Breite (bei 2-dimensionaler Betrachtung) und/oder einen gleichbleibenden oder im Wesentlichen gleichbleibenden Durchmesser (bei 3-dimensionaler Betrachtung) aufweist, mittels Thermoformung und/oder Thermofixierung nach dem Wirken mit unterschiedlichen Durchmessern und/oder Durchmessersprüngen, insbesondere entlang des Hauptmantelkörpers, versehen werden.

Nach der Formgebung und/oder Fixierung können erneut Parameter des Gefäßimplantats mit gemessenen Parametern des zu behandelnden Gefäßabschnittes und/oder bestimmten Parametern des Modells verglichen werden. So kann eine optimale Passgenauigkeit des patientenspezifischen Gefäßimplantats für den zu ersetzenden Gefäßabschnitt bestätigt werden.

In weiterer vorteilhafter Ausgestaltung des erfindungsgemäßen Verfahrens weist dieses ferner Schritte zur Veredelung des Gefäßimplantats (z.B. des endovaskulären Gefäßimplantats) auf, beispielsweise mittels "Coating" beziehungsweise der Aufbringung einer Schicht beispielsweise biokompatibler Gelatine und/oder Kollagen. Dies kann besonders vorteilhaft sein, wenn das Gefäßimplantat flüssigkeitsundurchlässig sein soll.

Ferner weist das erfindungsgemäße Verfahren in einer vorteilhaften Gestaltung bevorzugt einen Schritt des Sterilisierens des hergestellten Gefäßimplantats (z.B. des hergestellten endovaskulären Gefäßimplantats) auf.

In vorteilhafter Ausgestaltung des erfindungsgemäßen Verfahrens erfolgen die geschilderten Verfahrensschritte bevorzugt durch mindestens ein Computerprogramm. Sollte mehr als ein Computerprogramm genutzt werden, so weist das erfindungsgemäße Verfahren zusätzlich bei dem jeweiligen Wechsel des Computerprogramms die folgenden Schritte auf: optional Übersetzen der Informationen wie beispielsweise des Modells des Gefäßimplantats in ein Format, das von dem jeweiligen folgenden Computerprogramm lesbar ist, optional Speichern der Informationen in dem jeweiligen Format, Übergabe der Informationen in dem von dem jeweilig folgenden Computerprogramm lesbaren Format an das jeweilige Computerprogramm, und Lesen der erhaltenen Informationen durch das jeweilige Computerprogramm. Optional können zudem vor beziehungsweise nach einem oder mehreren der genannten Schritte ein oder mehrere Ausgaben an den Anwender vorgesehen sein, insbesondere Ausgaben wie Fehlermeldungen und/oder Abfragen bezüglich einer Speicherung der Informationen und/oder Übergabe der Informationen an das jeweilig folgende Computerprogramm. Insbesondere die Nutzung eines einzelnen Computerprogramms zumindest für die im Zusammenhang mit dem Messen und Bestimmen von Gefäßparametern sowie dem computergestützten Modell geschilderten Verfahrensschritte ist vorteilhaft, da so die Effizienz des Verfahrens maximiert werden kann.

Eine Abfrage, ob der Anwender zustimmt, mit dem erfindungsgemäßen Verfahren fortzufahren, kann nach jedem der beschriebenen Schritte des Verfahrens erfolgen. Ebenso kann ein Speichern der jeweiligen Daten während und/oder nach jedem der beschriebenen Schritte erfolgen.

Ein zweiter Aspekt der Erfindung betrifft ein Gefäßimplantat, bei dem es sich insbesondere um ein endovaskuläres Gefäßimplantat handeln kann. Das Gefäßimplantat ist vorzugsweise mit dem erfindungsgemäßen Verfahren gemäß dem ersten Erfindungsaspekt hergestellt. Das erfindungsgemäße Gefäßimplantat ist jedoch auf die Verwendung dieses Verfahrens nicht beschränkt. Insofern betrifft die Erfindung auch ein Gefäßimplantat mit einem der oben beschriebenen Strukturelemente, unabhängig davon, wie diese hergestellt werden. Die sich aus dem Verfahren gemäß dem ersten Erfindungsaspekt ergebenden Merkmale und Vorteile sind der Beschreibung des ersten Erfindungsaspektes zu entnehmen, wobei vorteilhafte Ausgestaltungen des ersten Erfindungsaspekts als vorteilhafte Ausgestaltungen des zweiten Erfindungsaspektes und umgekehrt anzusehen sind.

Im Folgenden werden einige besonders bevorzugte Ausführungsformen des erfindungsgemäßen Gefäßimplantats (z.B. des endovaskulären Gefäßimplantats) beispielhaft beschrieben, ohne den Schutzumfang der Erfindung zu beschränken. Angaben zu Strukturelementen der einzelnen im Folgenden geschilderten Ausführungsformen sind auch ohne explizite Nennung als Angaben zu jeweiligen Strukturelementen anderer Ausführungsformen zu verstehen. Für den Fachmann wird verständlich sein, dass die jeweiligen Strukturelemente in Abhängigkeit der Patientenanatomie vorzusehen sind und somit beliebige Kombinationen dieser Strukturelemente umfasst sind.

In einer vorteilhaften Ausgestaltung des erfindungsgemäßen Gefäßimplantats weist dieses mindestens eine Durchmesseränderung entlang mindestens eines tubulären Mantelkörpers eines jeweiligen Moduls auf. Eine Durchmesseränderung kann dabei ausgehend von einem bestimmten Ende des tubulären Hauptmantelkörpers als eine Verjüngung oder eine Aufweitung des tubulären Hauptmantelkörpers in dessen jeweiliger Haupterstreckungsrichtung ausgestaltet sein. So kann das Gefäßimplantat beispielsweise eine oder mehrere stufenartig ausgebildete Durchmesseränderungen aufweisen. Alternativ kann das Gefäßimplantat eine oder mehrere kontinuierlich ausgebildete Durchmesseränderungen aufweisen, die sich über mindestens 5 %, mindestens 10 %, mindestens 20 %, mindestens 30 %, mindestens 40 % oder auch mindestens 50 % der Länge des Gefäßimplantats in dessen Haupterstreckungsrichtung erstreckt bzw. erstrecken. Das Gefäßimplantat kann auch mehr als eine Durchmesseränderung entlang mindestens eines tubulären Mantelkörpers eines jeweiligen Moduls aufweisen, beispielsweise zwei, drei, vier oder fünf. Beispielsweise kann das Gefäßimplantat zwei Durchmesseränderungen aufweisen, wobei i) das Gefäßimplantat einen größeren Durchmesser in einem mittleren Abschnitt aufweist verglichen mit dem jeweiligen Durchmesser der terminalen Abschnitte, ii) das Gefäßimplantat einen kleineren Durchmesser in einem mittleren Abschnitt aufweist verglichen mit dem jeweiligen Durchmesser der terminalen Abschnitte, oder iii) das Gefäßimplantat einen mittleren Abschnitt aufweist, dessen Durchmesser einen Wert aufweist, der zwischen den jeweiligen Werten der Durchmesser der terminalen Abschnitte liegt.

In weiterer vorteilhafter Ausgestaltung des erfindungsgemäßen Gefäßimplantats weist dieses mindestens eine Bifurkation auf. So kann das Gefäßimplantat beispielsweise eine Bifurkation aufweisen, wobei die Bifurkation in einem mittleren Abschnitt oder in einem terminalen Abschnitt des Gefäßimplantats gelegen sein kann. So kann das Gefäßimplantat eine Bifurkation aufweisen, die so ausgestaltet ist, dass in Bezug auf die Haupterstreckungsrichtung des Gefäßimplantats der Abschnitt des tubulären Mantelkörpers des jeweiligen Moduls ohne Bifurkation beispielsweise bis zu 50 %, bis zu 60 %, bis zu 70 %, bis zu 80 %, bis zu 90 % oder bis zu 95 % der Länge des Gefäßimplantats entspricht. Ferner können die zwei Bifurkationsmantelkörper, in die sich der Hauptmantelkörper an der Bifurkation teilt, gemessen in deren jeweiliger Haupterstreckungsrichtung jeweils eine Länge von mindestens 0,5 cm, mindestens 1 cm, mindestens 2,5 cm, oder mindestens 5 cm aufweisen. Die mindestens zwei aus dem einen resultierenden Mantelkörper können dabei in deren Haupterstreckungsrichtung gemessen, dieselbe, eine vergleichbare oder unterschiedliche Längen aufweisen. Eine unterschiedliche Länge bezeichnet hierbei eine um mindestens 10 %, mindestens 20 %, oder auch mindestens 30 % unterschiedliche Länge gemessen an dem längeren der beiden beziehungsweise dem längsten der aus dem Hauptmantelkörper resultierenden Bifurkationsmantelkörper.

In weiterer vorteilhafter Ausgestaltung des erfindungsgemäßen Gefäßimplantats weist dieses mindestens eine Abzweigung entlang eines jeweiligen tubulären Mantelkörpers eines Moduls des Gefäßimplantats auf. So kann das Gefäßimplantat beispielsweise eine Abzweigung aufweisen, wobei die Abzweigung in einem mittleren Abschnitt oder in einem terminalen Abschnitt des Hauptmantelkörpers von diesem abzweigen kann. So kann das Gefäßimplantat eine Abzweigung aufweisen, die vom Hauptmantelkörper in Bezug auf die Haupterstreckungsrichtung des Gefäßimplantats in einer Distanz zu einem terminalen Ende des Hauptmantelkörpers abzweigt, die mindestens 5 %, mindestens 10 %, mindestens 20 % oder mindestens 30 % der Länge des Gefäßimplantats entspricht. Das Gefäßimplantat kann beispielsweise auch zwei oder mehr Abzweigungen aufweisen, wobei die Abzweigungen gleiche oder verschiedene Distanzen zu einem terminalen Ende des Hauptmantelkörpers gemessen in dessen Haupterstreckungsrichtung aufweisen können. Weist das Gefäßimplantat zwei oder mehr Abzweigungen auf, so können die Abzweigungen in Bezug auf die Haupterstreckungsrichtung des Hauptmantelkörpers von diesem in dem gleichen oder in verschiedenen Winkeln abgehen. Zudem können die mindestens zwei Abzweigungen von dem Hauptmantelkörper so abgehen, dass die jeweiligen Verzweigungsstellen in Bezug auf den Hauptmantelkörper auf einer Geraden angeordnet sind, die parallel zur Mittelachse des Hauptmantelkörpers in dessen Haupterstreckungsrichtung verläuft und/oder in Umfangsrichtung des Hauptmantelkörpers versetzt zueinander. Ferner kann die mindestens eine Abzweigung eine Länge des Seitmantelkörpers gemessen in dessen Haupterstreckungsrichtung von mindestens 0,3 cm, mindestens 0,5 cm, mindestens 1 cm, mindestens 2,5 cm, oder mindestens 5 cm aufweisen.

In weiterer vorteilhafter Ausgestaltung des erfindungsgemäßen Gefäßimplantats weist dieses mindestens eine Aussparung auf, wobei die mindestens eine Aussparung als mindestens ein Scallop und/oder mindestens eine Fenestrierung ausgebildet sein kann. Der mindestens eine Scallop weist bevorzugt eine in Strömungsrichtung gemessene Aussparungshöhe von 3 mm bis 20 mm auf, bevorzugt von 5 mm bis 15 mm. Der mindestens eine Scallop weist bevorzugt eine Aussparungsweite, vorzugsweise quer zur Strömungsrichtung oder in Umfangsrichtung gemessen, von 2 mm bis 15 mm, bevorzugt von 5 mm bis 12 mm, stärker bevorzugt von 7 mm bis 12 mm auf. Die mindestens eine Fenestrierungen ist bevorzugt oval oder rund ausgestaltet, besonders bevorzugt mit einem Verhältnis von Höhe zu Breite zwischen 0,5 und 2,5, bevorzugt zwischen 0,8 und 2, besonders bevorzugt zwischen 1 und 1,35. Die mindestens eine Fenestrierung ist bevorzugt in einem mittleren Bereich des Gefäßimplantats ausgestaltet, beispielsweise in einer Distanz zu einem Ende des Gefäßimplantats, die mindestens 5 %, mindestens 10 % oder mindestens 30 % der Länge des Gefäßimplantats entspricht. Alternativ oder optional ist die mindestens eine Fenestrierung bevorzugt so ausgestaltet, dass sie mindestens 2 mm, mindestens 5 mm oder mindestens 7 mm zu dem nächstgelegenen Ende des Gefäßimplantats entfernt ist. Das Gefäßimplantat kann auch zwei oder mehr Aussparungen aufweisen, beispielsweise eine Fenestrierung und einen Scallop, zwei Fenestrierungen, zwei Scallops, zwei Fenestrierungen und einen Scallop, eine Fenestrierung und zwei Scallops, drei Fenestrierungen, drei Scallops, drei Fenestrierungen und einen Scallop, oder auch zwei Fenestrierungen und zwei Scallops. Weist das Gefäßimplantat zwei oder mehr Aussparungen auf, so können deren jeweilige Positionen in Bezug auf den tubulären Mantelkörper in einer Geraden angeordnet sein, die parallel zur Mittelachse des Mantelkörpers in dessen Haupterstreckungsrichtung verläuft und/oder in Umfangsrichtung des Mantelkörpers versetzt zueinander und/oder über den Umfang des Mantelkörpers verteilt, aber auf gleicher Position entlang der Längsachse des Mantelkörpers.

In weiterer vorteilhafter Ausgestaltung des erfindungsgemäßen Gefäßimplantats weist dieses mindestens eine lokale Verstärkung auf. Bevorzugt weist das Gefäßimplantat lokale Verstärkungen in Bereichen auf, an denen sich der Mantelkörper in zwei Bifurkationsmantelkörper oder in einen Hauptmantelkörper und einen Seitmantelkörper aufteilt, und/oder in Bereichen des Gewirks, die eine Aussparung begrenzen, insbesondere in Wirkrichtung terminale Bereiche eines Scallops und/oder einer Fenestrierung. Im Falle einer Fenestrierung weisen bevorzugt beide in Wirkrichtung terminalen Bereiche eine lokale Verstärkung auf. Im Falle eines Scallops weist bevorzugt mindestens der Bereich, der die größte Distanz zu einem Ende des Gefäßimplantats befindet, eine lokale Verstärkung auf. Die Verstärkung kann insbesondere durch eine räumlich begrenzte Bindungsänderung, insbesondere eine räumlich begrenzte Köperbindung erfolgen.

In weiterer vorteilhafter Ausgestaltung des erfindungsgemäßen Gefäßimplantats weist dieses mindestens eine Durchmesseränderung und mindestens eine lokale Verstärkung auf, wobei die mindestens eine lokale Verstärkung sich in einem Abschnitt einer Durchmesseränderung befinden kann oder auch nicht.

In weiterer vorteilhafter Ausgestaltung des erfindungsgemäßen Gefäßimplantats weist dieses mindestens eine Bifurkation und/oder Abzweigung und mindestens eine lokale Verstärkung auf, wobei die mindestens eine lokale Verstärkung sich in der mindestens einen Bifurkation und/oder Abzweigung befinden kann, in dem Bereich des Übergangs vom Mantelkörper in die mindestens eine Bifurkation und/oder Abzweigung oder auch in anderen Bereichen des Mantelkörpers.

In weiterer vorteilhafter Ausgestaltung des erfindungsgemäßen Gefäßimplantats weist dieses mindestens eine Aussparung und mindestens eine lokale Verstärkung auf, wobei mindestens eine Aussparung mit mindestens einer lokalen Verstärkung ausgebildet ist. Die besonders bevorzugten Bereiche der lokalen Verstärkungen bei Fenestrierung beziehungsweise Scallop sind wie oben bereits angegeben zu verstehen. Das Gefäßimplantat kann beispielsweise mindestens eine Aussparung ohne lokale Verstärkung sowie mindestens eine Aussparung mit mindestens einer lokalen Verstärkung wie zuvor beschrieben aufweisen. Bevorzugt weisen alle der mindestens einen Aussparung eine jeweilige lokale Verstärkung auf. Optional kann das Gefäßimplantat lokale Verstärkungen in weiteren Bereichen aufweisen.

In weiterer vorteilhafter Ausgestaltung des erfindungsgemäßen Gefäßimplantats weist dieses mindestens eine Durchmesseränderung und mindestens eine Bifurkation auf, wobei die mindestens eine Bifurkation sich in einem Abschnitt einer Durchmesseränderung befinden kann oder auch nicht. Beispielsweise kann das Gefäßimplantat mindestens eine Durchmesseränderung und eine Bifurkation aufweisen, wobei sich die mindestens eine Durchmesseränderung in dem Mantelkörper befinden kann und/oder in mindestens einem der beiden durch die Bifurkation resultierenden Mantelkörper. Optional kann das Gefäßimplantat lokale Verstärkungen in Bereichen wie zuvor bereits geschildert aufweisen und/oder in weiteren Bereichen des Gefäßimplantats.

In weiterer vorteilhafter Ausgestaltung des erfindungsgemäßen Gefäßimplantats weist dieses mindestens eine Durchmesseränderung und mindestens eine Abzweigung auf, wobei die mindestens eine Abzweigung sich in einem Abschnitt einer Durchmesseränderung befinden kann oder auch nicht. Beispielsweise kann das Gefäßimplantat mindestens eine Durchmesseränderung und eine Abzweigung aufweisen, wobei sich die mindestens eine Durchmesseränderung in dem mindestens einen Hauptmantelkörper befinden kann und/oder in dem mindestens einen Seitmantelkörper. Optional kann das Gefäßimplantat lokale Verstärkungen in Bereichen wie zuvor bereits geschildert aufweisen und/oder in weiteren Bereichen des Gefäßimplantats.

In weiterer vorteilhafter Ausgestaltung des erfindungsgemäßen Gefäßimplantats weist dieses mindestens eine Durchmesseränderung und mindestens eine Aussparung auf, wobei die mindestens eine Aussparung sich in einem Abschnitt einer Durchmesseränderung befinden kann oder auch nicht. Hierbei können die mindestens eine Durchmesseränderung und/oder die mindestens eine Aussparung mindestens eine lokale Verstärkung aufweisen, insbesondere die mindestens eine Aussparung. Das Gefäßimplantat kann beispielsweise mindestens eine Aussparung ohne lokale Verstärkung sowie mindestens eine Aussparung mit mindestens einer lokalen Verstärkung wie zuvor beschrieben aufweisen.

In weiterer vorteilhafter Ausgestaltung des erfindungsgemäßen Gefäßimplantats weist dieses mindestens eine Bifurkation und mindestens eine Abzweigung auf. Hierbei können die mindestens eine Bifurkation und/oder die mindestens eine Abzweigung mindestens eine lokale Verstärkung aufweisen.

In weiterer vorteilhafter Ausgestaltung des erfindungsgemäßen Gefäßimplantats weist dieses mindestens eine Bifurkation und/oder Abzweigung und mindestens eine Auspaarung auf, wobei mindestens eine Aussparung bevorzugt mit mindestens einer lokalen Verstärkung ausgebildet ist. Bevorzugt weist der Hauptmantelkörper des Gefäßimplantats die mindestens eine Aussparung auf, doch können optional auch einer der durch eine Bifurkation resultierenden Bifurkationsmantelkörper und/oder Seitmantelkörper mindestens eine Aussparung aufweisen, bevorzugt mit jeweils mindestens einer lokalen Verstärkung. Das Gefäßimplantat kann beispielsweise mindestens eine Aussparung ohne lokale Verstärkung sowie mindestens eine Aussparung mit mindestens einer lokalen Verstärkung wie zuvor beschrieben aufweisen.

In weiterer vorteilhafter Ausgestaltung des erfindungsgemäßen Gefäßimplantats weist dieses mindestens eine Durchmesseränderung, mindestens eine Bifurkation und mindestens eine Abzweigung auf, wobei die mindestens eine Bifurkation und/oder die mindestens eine Abzweigung sich in einem Abschnitt einer Durchmesseränderung befinden kann oder auch nicht. Beispielsweise kann das Gefäßimplantat mindestens eine Durchmesseränderung, eine Bifurkation und eine Abzweigung aufweisen, wobei sich die mindestens eine Durchmesseränderung in dem Mantelkörper, in mindestens einem der durch die Bifurkation resultierenden Bifurkationsmantelkörper, und/oder in dem mindestens einen Seitmantelkörper befinden kann. Optional kann das Gefäßimplantat lokale Verstärkungen in Bereichen wie zuvor bereits geschildert aufweisen und/oder in weiteren Bereichen des Gefäßimplantats.

In weiterer vorteilhafter Ausgestaltung des erfindungsgemäßen Gefäßimplantats weist dieses mindestens eine Durchmesseränderung, mindestens eine Bifurkation und/oder Abzweigung und mindestens eine Aussparung auf, wobei insbesondere mindestens eine Aussparung bevorzugt mit mindestens einer lokalen Verstärkung ausgebildet ist. Bevorzugt weist der tubuläre Mantelkörper des Gefäßimplantats die mindestens eine Aussparung auf, doch können optional auch einer der durch eine Bifurkation resultierenden Mantelkörper und/oder Seitmantelkörper mindestens eine Aussparung aufweisen, bevorzugt mit jeweils mindestens einer lokalen Verstärkung. Das Gefäßimplantat kann beispielsweise mindestens eine Aussparung ohne lokale Verstärkung sowie mindestens eine Aussparung mit mindestens einer lokalen Verstärkung wie zuvor beschrieben aufweisen.

Ein erfindungsgemäßes Gefäßimplantat, z.B. ein endovaskuläres Gefäßimplantat, kann beispielsweise als Stentgraft ausgebildet sein. Der hier verwendete Begriff "Stentgraft" bezeichnet vorzugsweise eine Stentstruktur mit einem daran befestigten Graft und umfasst somit jede Art von Gefäßimplantaten, insbesondere von endovaskulären Gefäßimplantaten, die zumindest temporär mindestens eine vorzugsweise tubuläre Struktur mit einer Mehrzahl von Streben und mindestens eine vorzugsweise polymerbasierte Materialschicht als Ummantelungskomponente aufweisen. Der Stentgraft kann zur Behandlung von Aneurysmen ausgebildet sein. Insbesondere kann es sich bei einem erfindungsgemäßen Stentgraft um einen Stentgraft zur Behandlung von Aortenaneurysmen handeln, wie etwa zur Behandlung von Aneurysmen der thorakalen, thorako-abdominalen oder abdominalen Aorta. Die hergestellte textile und/oder textilbasierte Struktur kann dabei den Graft bilden. Der Stent kann gemäß einem beliebigen Verfahren an diesem Graft befestigt werden, vorzugsweise jedoch nach der Herstellung des Graft mittels der beschriebenen additiven und/oder generativen Verfahren.

Die Stentstruktur weist vorzugsweise eine Mehrzahl von miteinander verbundenen Streben auf, wobei die Streben in einem Querschnitt senkrecht zur Verlaufsrichtung der jeweiligen Strebe in Radialrichtung des Stentgrafts eine Höhe von mindestens 0,01 mm, vorzugsweise mindestens 0,05 mm oder mindestens 0,1 mm haben. So können die Streben zum Beispiel eine Höhe von 0,01 mm bis 3 mm, bevorzugt von 0,05 mm bis 1 mm aufweisen. Alternativ oder zusätzlich können die Streben in diesem Querschnitt senkrecht zur Höhe eine Breite von mindestens 0,01 mm, vorzugsweise mindestens 0,05 mm oder 0,1 mm haben. So können die Streben zum Beispiel eine Breite von 0,01 mm bis 3 mm, bevorzugt von 0,05 mm bis 1 mm aufweisen. Insbesondere im Fall drahtbasierter Streben und/oder Drahtstrukturen als Stentstruktur können die Streben und/oder Drahtstrukturen in einem Querschnitt senkrecht zur jeweiligen Verlaufsrichtung einen Durchmesser von mindestens 0, 1 mm, vorzugsweise von mindestens 0, 3 mm oder 0, 5 mm aufweisen.

Die Streben können in dem Fachmann bekannten Ausgestaltungsformen angeordnet sein, beispielsweise in zickzack- und/oder mäanderförmigen Ringen (Kronen), spiralförmig und/oder in zickzackförmigen Spiralen. Die Spiralen und/oder Ringe können ihrerseits über Verbinder miteinander verbunden sein. Die Streben können so vorgesehen sein, dass sie im expandierten Zustand höchstens 50 %, höchstens 40 %, höchstens 30 %, höchstens 20 %, höchstens 12 %, höchstens 10 %, höchstens 8 %, höchstens 6 %, höchstens 5 % oder höchstens 3 % der äußeren Mantelfläche des Grafts bedecken. Ferner können die Streben so vorgesehen sein, dass sie im expandierten Zustand mindestens 12 %, mindestens 10 %, mindestens 8 %, mindestens 5 %, mindestens 3 % oder mindestens 1 % der äußeren Mantelfläche des Grafts bedecken.

Bevorzugte Ausführungsformen der Erfindung werden nachfolgend unter Bezugnahme auf die Zeichnungen beispielhaft beschrieben. Es handelt sich hierbei lediglich um schematische Darstellungen, die zur Verdeutlichung bestimmter Aspekte andere (optionale) Strukturen gegebenenfalls nicht darstellen oder aber auch verschiedene optionale, miteinander einhergehende Aspekte in einer Darstellung berücksichtigen. Gleiche Bezugszeichen weisen auf äquivalente, ähnliche, vergleichbare oder gleiche Elemente in den dargestellten Ausführungsformen hin.

Die gezeigten Ausführungsformen können innerhalb des Schutzumfangs der Ansprüche in vielerlei Hinsicht verändert werden. Die Offenbarung der Figuren soll den Schutzumfang der Erfindung nicht beschränken. Dabei ist zu beachten, dass die Merkmale der obengenannten Ausführungsformen in einer einzigen Ausführungsform kombiniert werden können. Ausführungsformen der Erfindung können daher je nach Ausgestaltung alle oder nur einige der obengenannten Merkmale aufweisen. Es zeigen:
- Fig. 1: ein schematisches Ablaufdiagramm eines erfindungsgemäßen Verfahrens;
- Fig. 2: eine schematische Darstellung eines Gefäßimplantats mit verschiedenen Strukturelementen;
- Fig. 3: eine schematische Darstellung des integralen Wirkens einer Abzweigung schräg zur Haupterstreckungsachse des Gefäßimplantats;
- Fig. 4: eine schematische Darstellung des integralen Wirkens einer Abzweigung im Wesentlichen parallel zur Haupterstreckungsachse des Gefäßimplantats;
- Fig. 5: eine schematische Darstellung einer Durchmesseränderung im Bereich eines Iliakalgefäß-Grafts;
- Fig. 6: eine schematische Darstellung einer Fenestrierung mit lastgerechter Bindungsänderung;
- Fig. 7: eine schematische Darstellung des Wirkens eines Scallop mit Trennfäden;
- Fig. 8: eine schematische Darstellung eines Gewirks mit und ohne Unterlegung;
- Fig. 9: eine schematische Darstellung des integralen Wirkens einer Abzweigung quer zur Haupterstreckungsachse des Gefäßimplantats;
- Fig. 10: eine beispielhafte Bindungsabfolge beim Wirken der Abzweigung von Fig. 9 (Vorderansicht);
- Fig. 11: die Bindungsabfolge von Fig. 10 schräg von der Seite;
- Fig. 12: die Bindungsabfolge von Fig. 10 in Seitenansicht;
- Fig. 13: eine weitere schematische Darstellung des integralen Wirkens einer Abzweigung schräg zur Haupterstreckungsachse des Gefäßimplantats;
- Fig. 14: eine schematische Darstellung des integralen Wirkens einer Durchmesseränderung des Gefäßimplantats.

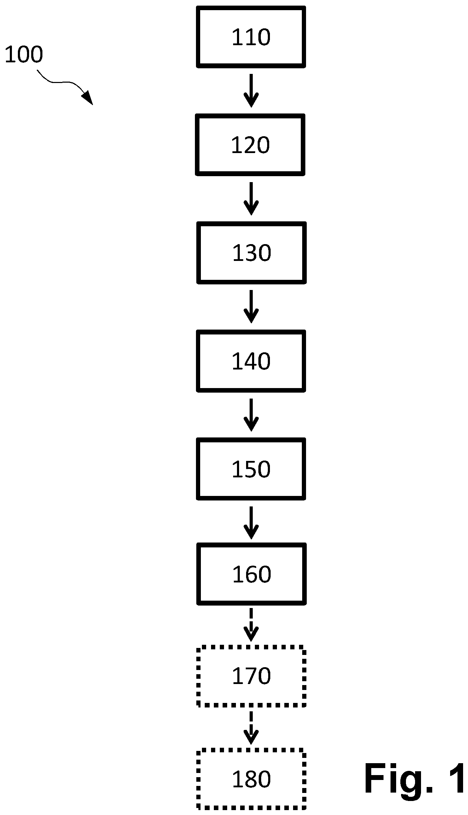

**Figur** 1 zeigt als schematisches Ablaufdiagramm die Schritte eines erfindungsgemäßen Verfahrens 100 zur Herstellung eines Gefäßimplantats, bei dem es sich z.B. um ein endovaskuläres Gefäßimplantat 1 handeln kann.

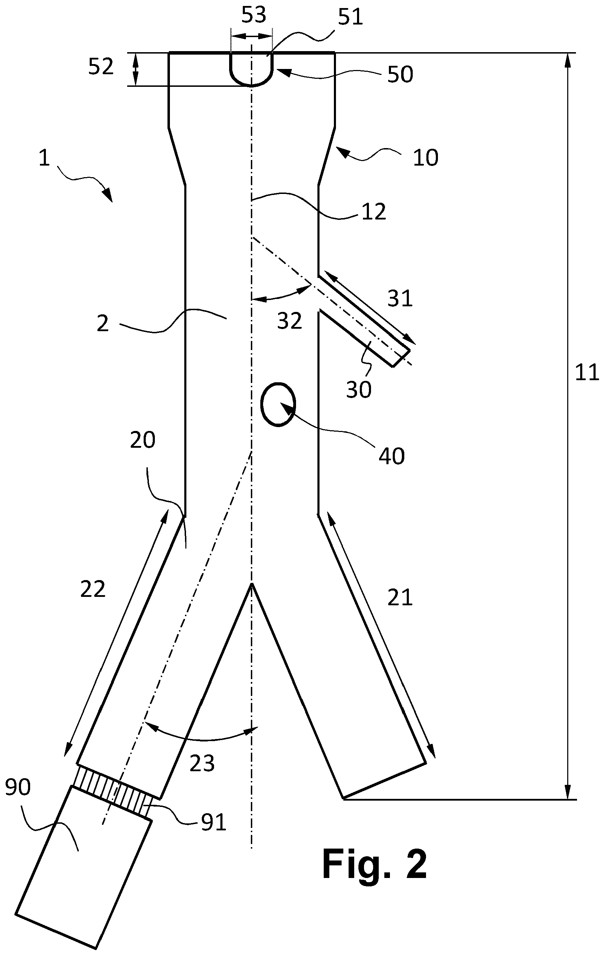

In einer bevorzugten Ausführungsform des Verfahrens werden zunächst Gefäßparameter erhalten (Schritt 110). Diese Gefäßparameter wurden bevorzugt anhand pre-operativer medizinischer Bilddaten eines Gefäßes erhalten, die beispielsweise im Zuge einer Computer- und/oder Magnetresonanztomographie gewonnen wurden. Die patientenspezifischen Bilddaten können auf verschiedene, dem Fachmann bekannte Weise bearbeitet werden, um eine möglichst hohe, bevorzugt 3-dimensionale, Auflösung zu erhalten. Um die Gefäßparameter zu erhalten werden die Bilddaten bevorzugt visualisiert, der Gefäßabschnitt, für den ein endovaskuläres Gefäßimplantat **1** (siehe **Figur 2**) hergestellt werden soll, identifiziert und die jeweiligen Gefäßparameter gemessen wie beispielsweise Position, Durchmesser und Ausdehnung des zu überbrückenden und/oder zu ersetzenden Gefäßabschnittes, sowie die Lage und Geometrie anatomischer Besonderheiten, wie etwa Abzweigungen und Bifurkationen des Gefäße erfasst.

Erhaltene Gefäßparameter (Schritt **110**) werden gemäß einer bevorzugten Ausführungsform des Verfahrens **100** zur Erstellung eines computergestützten Modells des endovaskulären Gefäßimplantats **1** herangezogen (Schritt **120**). Das computergestützte Modell des Gefäßimplantats **1** kann beispielsweise für eine erleichterte Bedienung sowie zur optischen Kontrolle visualisiert werden.

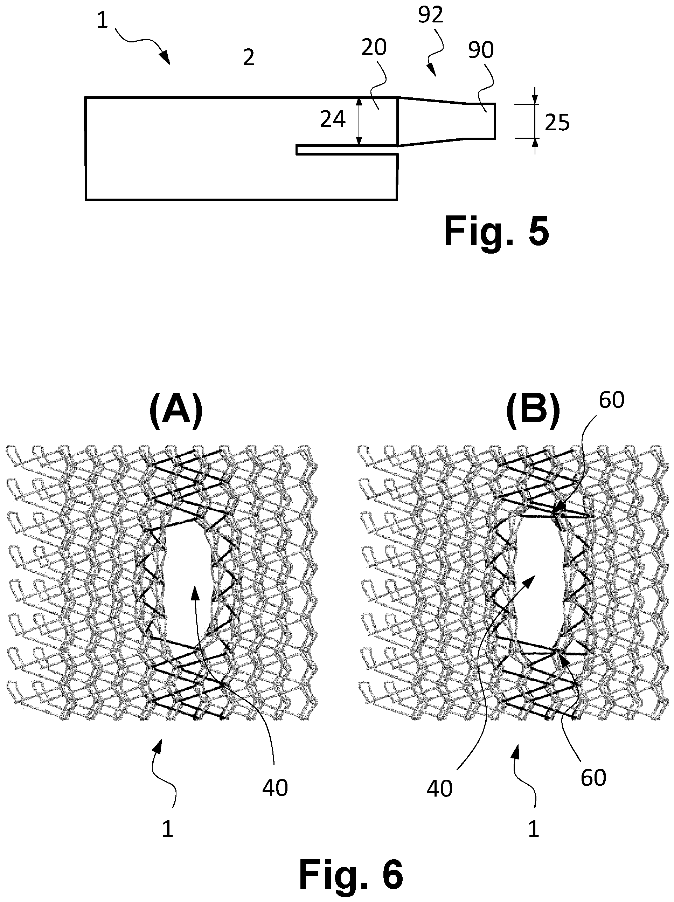

Um eine optimale Passform des Gefäßimplantats **1** nach dessen Einbringung in das jeweilige Gefäß zu gewährleisten, wird daraufhin abgefragt, ob ein oder mehrere Strukturelemente hierfür relevant sind (Schritt **130**). Derartige Strukturelemente können beispielsweise eine oder mehrere Durchmesseränderungen **10** entlang eines tubulären Hauptmantelkörpers **2** des Gefäßimplantats **1,** eine oder mehrere Bifurkationen **20,** eine oder mehrere Abzweigungen **30** entlang eines tubulären Mantelkörpers **2** des Gefäßimplantats **1,** eine oder mehrere Aussparungen **40, 50** entlang des Mantelkörpers **2,** und/oder eine oder mehrere lokale Verstärkungen **60** sein (siehe **Figur 2** und **Figur 5****).** Die Abfrage kann manuell, beispielsweise mittels einer Eingabemaske, oder automatisiert erfolgen, beispielsweise durch einen Abgleich mit in einer entsprechenden Datenbank gespeicherten jeweiligen Gefäßparametern. Erfolgt eine positive Abfrage zu mindestens einem der Strukturelemente, so werden die jeweiligen Parameter bestimmt (Schritt **140**). Auch dies kann durch manuelle Eingabe umgesetzt werden, bevorzugt erfolgt die Bestimmung jedoch automatisch oder teilweise automatisch durch ein jeweiliges Computerprogramm. Die jeweiligen Strukturelemente werden daraufhin basierend auf den jeweiligen bestimmten Parametern in das computergestützte Modell (Schritt **150**) integriert. Das computergestützte Modell kann mindestens eine technische Zeichnung, Designdatei, JC Datei und/oder KMO Datei sein, wobei bevorzugt ein digitales 3D-Modell erstellt und direkt oder indirekt in eine maschinenlesbare KMO Datei umgewandelt wird. Auch eine automatisierte Umwandlung der Modelldaten in Maschinendaten ist möglich.

Basierend auf dem erstellten computergestützten Modell erfolgt schließlich die Herstellung (Schritt **160**) des erfindungsgemäßen, textilen und/oder textilbasierten sowie bevorzugt individualisierten Gefäßimplantats **1** mittels Jacquard-Technik anhand der erstellten, von einer jeweiligen Wirkmaschine lesbaren Datei, bevorzugt einer KMO Datei. Anschließend kann optional ein Thermoformen und/oder Thermofixieren des Gefäßimplantats 1 (Schritt **170**) und/oder ein Aufbringen einer Stentstruktur (Schritt **180**) erfolgen.

**Figur 2** zeigt die schematische Darstellung eines erfindungsgemäßen Gefäßimplantats **1** mit verschiedenen Strukturelementen. Das hier beispielhaft dargestellte Gefäßimplantat **1** ist als Graft ausgebildet und weist einstückig mit dem Mantelkörper des Graft ausgebildete Strukturelemente auf. Diese sind wie hier gezeigt eine Durchmesseränderung **10** entlang des tubulären Mantelkörpers **2** des Gefäßimplantats **1,** eine Bifurkation **20,** eine Abzweigung **30** entlang des tubulären Mantelkörpers **2** des Gefäßimplantats **1,** sowie zwei Auspaarungen (**40, 50**), wobei eine Auspaarung als Fenestrierung **40** und eine Aussparung als Scallop **50** ausgebildet ist. Der Scallop **50** hat ein offenes Ende **51.**

Wie ferner dargestellt kann das Gefäßimplantat **1** entlang seiner Haupterstreckungsrichtung **12** eine Länge (d.h. Gesamtlänge) **11** aufweisen. Die Bifurkationen **20** können eine jeweilige Bifurkationslänge **21, 22** aufweisen. Der Seitenmantelkörper der Abzweigung **30** kann eine Länge **31** aufweisen, gemessen vom Ursprung am Hauptmantelkörper **2.** **Figur 2** zeigt ferner, dass die Bifurkationsmantelkörper vorzugsweise jeweils in einem Winkel **23** (nur für den Mantelkörper **22** gezeigt) zum Hauptmantelkörper angeordnet sind. Der Seitenmantelkörper ist vorzugsweise in einem Winkel **32** angeordnet. Die Winkel **23** betragen beispielsweise 0 bis 75, vorzugsweise 0 bis 45. Nach individueller Patientenanatomie können die Winkel **23** innerhalb der genannten Bereiche auch unterschiedlich sein. Der Winkel **32** beträgt vorzugsweise 0 bis 180, insbesondere 2 bis 170. Der Scallop **50** hat eine Aussparungshöhe **51** von 3 bis 20 mm, vorzugsweise von 5 bis 15 mm, und/oder eine Aussparungsbreite **53** von 2 bis 15 mm, vorzugsweise von 7 bis 12 mm.

Im Bereich einer Bifurkation **20** können beim Wirken des Gefäßimplantats **1** ein oder zwei Iliakalgefäß-Grafts **90** ausgebildet werden. Da Iliakalgefäß-Grafts **90** üblicherweise separat vom tubulären Mantelkörper **2** in den Körper eingeführt werden, sind die Iliakalgefäß-Grafts **90** vorzugsweise beim Wirken über Trennfäden **91** mit einem jeweiligen Ende der Bifurkation **20** verbunden, sodass die Iliakalgefäß-Grafts **90** nach Abschluss des Wirkens vom restlichen tubulären Mantelkörper **2** und der Bifurkation **20** getrennt werden können. Der oder die Iliakalgefäß-Grafts **90** sind dabei vorzugsweise je nach anatomischen Erfordernissen des konkreten Patienten mit einem gewünschten Durchmesserverlauf ausgebildet, etwa einem gewünschten konstanten Durchmesser oder mit einem oder mehreren Durchmessersprüngen oder Durchmesseränderungsverläufen. Entsprechende Durchmesseränderungen können, wie vorgehend beschrieben, durch eine Thermoformung erreicht werden, alternativ oder zusätzlich auch durch die Verwendung von weniger bzw. zusätzlichen Fäden und/oder die Wahl der Parameter. Die Iliakalgefäß-Grafts können je nach patientenspezifischen anatomischen Erfordernissen mindestens eine Abzweigung und/oder mindestens eine Aussparung beinhalten.

Die abgebildeten Strukturelemente sind rein exemplarisch zu verstehen. Es können daher je nach Ausgestaltung des Gefäßimplantats **1** ein oder mehrere der genannten Strukturelemente ausgebildet sein, ein oder mehrere einer jeden Art an Strukturelement, sowie an verschiedenen Positionen in Bezug auf den Mantelkörper des Gefäßimplantats **1** wie oben geschildert. Bevorzugt ist das mindestens eine Strukturelement jeweils patientenspezifisch ausgestaltet.

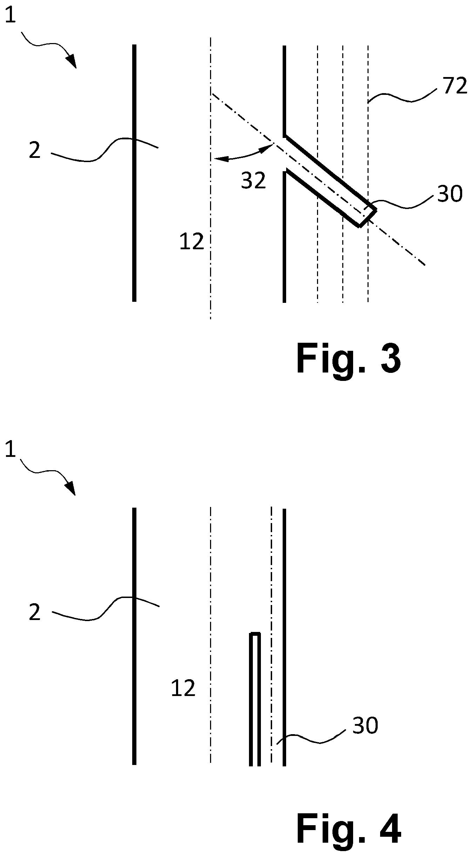

**Figur 3** zeigt ein Detail von **Figur 2** und insbesondere schematisch eine integrale Ausgestaltung des Seitmantelkörpers an der Abzweigung **30** schräg zur Haupterstreckungsachse **12** des Gefäßimplantats **1.** Wie in **Figur 3** dargestellt ist, werden zur schrägen Ausbildung der Abzweigung im Wirkprozess **30** vorzugsweise Trennfäden **72** eingesetzt, vorzugsweise Maschenstäbchen ohne Unterlegung, die nach dem Abschluss des Wirkverfahrens abgetrennt werden (z.B. durch mechanisches Schneiden oder Laserschneiden). Der Winkel **32,** in dem die Abzweigung **30** vom Hauptmantelkörper **2** absteht, kann nach Abschluss des Wirkverfahrens in einem optionalen Schritt **170** des Thermoformens und/oder Thermofixierens (siehe **Figur 1****)** bei Bedarf weiter angepasst werden.

**Figur 4** zeigt schematisch eine weitere integrale Ausgestaltung des Seitmantelkörpers an der Abzweigung **30** mit dem Hauptkörper **2.** In diesem Fall wird der Seitmantelkörper im Wesentlichen parallel oder parallel zur Haupterstreckungsachse **12** des Gefäßimplantats **1** gewirkt. Nach Abschluss des Wirkverfahrens kann die Winkelposition des Seitmantelkörpers optional durch einen Schritt **170** des Thermoformens und/oder Thermofixierens (siehe **Figur 1****)** an das zu behandelnde Gefäß angepasst werden. Bei dieser im Wesentlichen parallelen oder parallelen Wirkung des Seitmantelkörpers zur Haupterstreckungsachse **12** kann auf Trennfäden verzichtet werden. Optional können jedoch auch in diesem Zusammenhang Trennfäden eingesetzt werden.

Auch wenn in **Figuren 3 und 4** jeweils die Herstellung einer Abzweigung **30** gezeigt ist, können die entsprechenden Verfahren ebenso zur Herstellung einer Bifurkation **20** von **Figur 2** verwendet werden.

**Figur 5** zeigt das Gefäßimplantat **1** mit einer Bifurkation **20,** wobei am Ende der Bifurkation **20** ein Iliakalgefäß-Graft **90** vorgesehen ist. Wie hierbei schematisch dargestellt ist, kann die Bifurkation **20** an ihrem vom restlichen Hauptmantelkörper **2** abgewandten Ende - also an dem Ende, das dem Iliakalgefäß-Graft zugewandt ist **-** einen Durchmesser **24** aufweisen. Da dieser Durchmesser **24** aus herstellungsbedingten Gründen, und/oder um die Implantation im Patienten zu erleichtern, größer als der Durchmesser des Iliakalgefäßes und/oder größer als ein gewünschter Durchmesser **25** an einem stromabwärtsseitigen Ende des Iliakalgefäß-Grafts **90** gewählt werden kann, kann es vorteilhaft sein, entlang des Iliakalgefäß-Grafts **90** mindestens eine Durchmesseränderung **92** vorzusehen. Auf diese Weise kann der Durchmesser des Iliakalgefäß-Grafts **90** auf den für das Iliakalgefäß gewünschten Durchmesser **25** reduziert werden. Der Durchmesser des Iliakalgefäß-Grafts **90** nimmt in **Figur 5** in Richtung des Blutflusses ab. Der Durchmesser des Iliakalgefäß-Grafts **90** könnte in Richtung des Blutflusses jedoch auch zunehmen. Der Durchmesser **24** des Hauptmantelkörpers **2** könnte bei Bedarf daher auch kleiner als der Durchmesser **25** an dem Ende des Iliakalgefäß-Graft **90** sein, das vom Hauptmantelkörper **2** abgewandt ist. Für den Fachmann wird zudem erkennbar sein, dass entsprechende Gestaltungen auch an anderen Körperstellen Verwendung finden können. Bei dem oder den Grafts **90** kann es sich daher auch um Grafts für andere Gefäße handeln.

**Figur 6** zeigt schematisch eine Fenestrierung **40** ohne (A) beziehungsweise mit (B) lokaler Verstärkung **60** durch eine lastgerechte Bindungsänderung durch den Einsatz einer Köperbindung **60.** In schwarz ist der Fadenverlauf ohne (A) beziehungsweise mit (B) Köperbindung **60** dargestellt. Wie im Vergleich von (A) und (B) zu sehen, erlaubt die hier beispielhaft dargestellte Köperbindung **60** die Aufteilung der Zuglast von einem Faden (A) auf zwei in Umfangrichtung verlaufende Fäden (B).

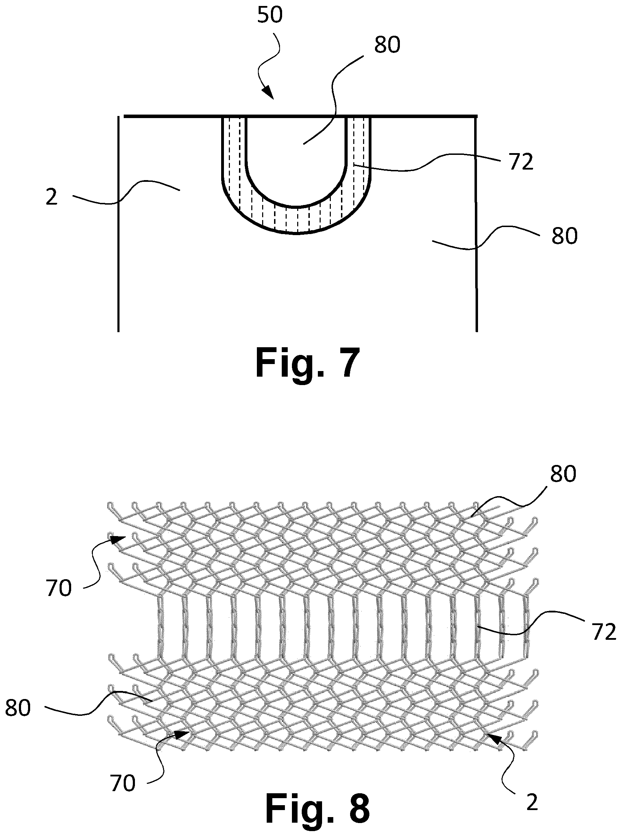

**Figur 7** zeigt schematisch eine mögliche Ausgestaltung des Scallop **50** von **Figur 2****.** Hierbei werden Trennfäden **72,** also vorzugsweise Maschenstäbchen ohne Unterlegung, zwischen einem Abschnitt mit Unterlegung **80** und dem restlichen (mit Unterlegung gewirkten) Hauptmantelkörper **2** vorgesehen. Die Trennfäden **72** werden anschließend abgetrennt, sodass der Abschnitt mit Unterlegung aus dem Scallop **50** entnommen wird und der Scallop **50** eine Öffnung im Hauptmantelkörper **2** bildet. Alternativ kann der Abschnitt mit Unterlegung **80** auch durch längere Trennfäden **72** ersetzt werden. Der Abschnitt mit Unterlegung **80** kann hierbei auch als eine Grundbindung **70** bezeichnet werden

**Figur 8** zeigt eine schematische Detailansicht des Abschnitts mit Unterlegung **80** und dessen Verbindung mit dem (mit Unterlegung gewirkten) Hauptmantelkörper **2.** Wie gezeigt, können die Trennfäden **72** im Rahmen der vorliegenden Erfindung insbesondere als Maschenstäbchen ohne Unterlegung ausgebildet sein.

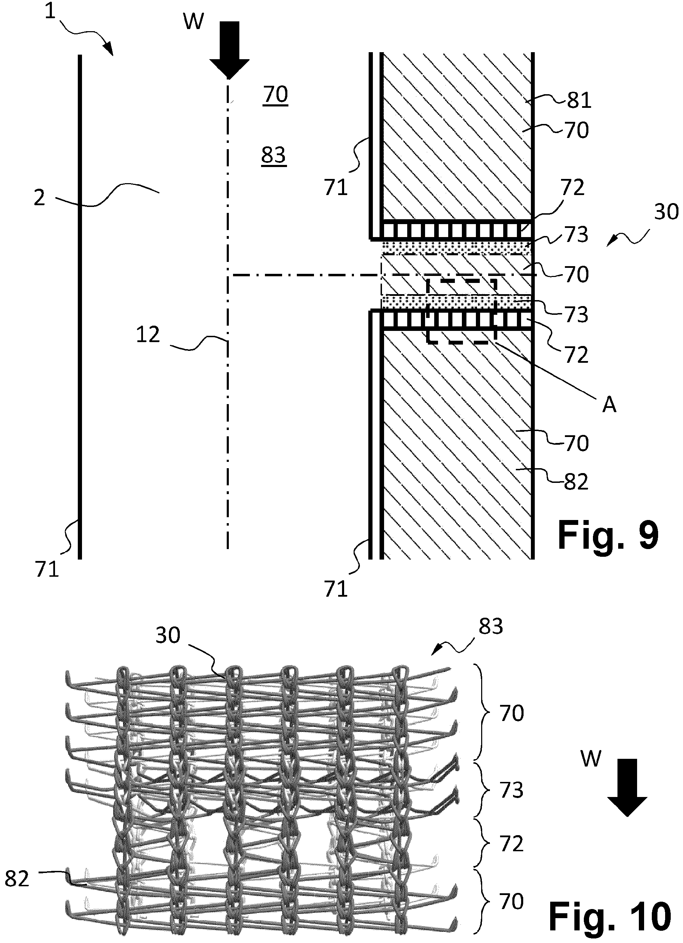

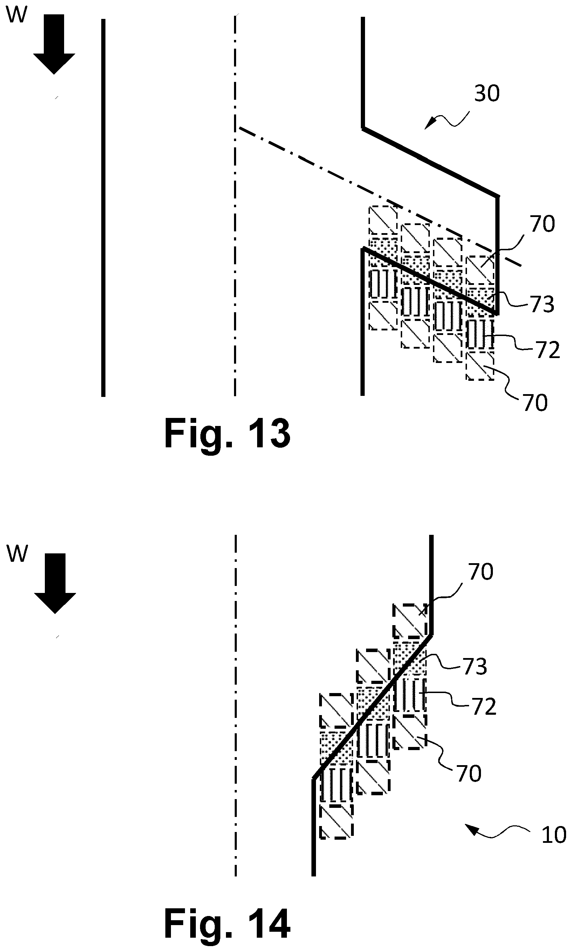

**Figur 9** zeigt beispielhaft eine weitere mögliche Bindungsabfolge bei einem integralen Wirken verschiedener Strukturelemente des Gefäßimplantats **1,** hier beispielhaft an einer Abzweigung **30** dargestellt, die im Wesentlichen senkrecht zur Haupterstreckungsrichtung **12** des Hauptmantelkörpers **2** angeordnet ist (wobei dies lediglich der besseren Veranschaulichung dient und eine entsprechende Bindungsabfolge auch im Zusammenhang mit anderen Anordnungen der Abzweigung **30** oder anderen Strukturelementen eingesetzt werden kann, siehe auch **Figuren 13 und 14****).**

Der Hauptmantelkörper **2** wird hierbei mit einer vorderen Textilfläche **83** und einer hinteren Textilfläche **84** (in **Figur 9** nicht sichtbar) gewirkt, die beide eine Grundbindung **70** aufweisen. Die Grundbindungen **70** der vorderen und hinteren Textilflächen können optional übereinstimmen.

Die Grundbindungen **70** der vorderen Textilfläche **83** und der hinteren Textilfläche **84** sind entlang mindestens eines Randbereichs (in **Figur 9** entlang zwei Randbereichen) durch eine Randabbindung **71** miteinander verbunden, woraus der tubuläre Hauptmantelkörper **2** resultiert. Randabbindungen können insbesondere dann eingesetzt werden, wenn die vordere und die hintere Textilfläche **83, 84** parallel zur Produktionsrichtung **W** verbunden werden. Die Fäden zur Herstellung der Abzweigung **30** laufen bei der Herstellung des Hauptmantelkörpers **2** mit, wie auch bereits in **Figur 3** dargestellt, sind in der Variante von **Figur 9** jedoch abschnittsweise als Grundbindung **70** ausgebildet (in **Figuren 9****,** **13 und 14** schematisch mit einer schrägen Schraffur dargestellt). Hierdurch lässt sich eine bessere Prozessstabilität des Wirkprozesses erreichen. Es ergibt sich ein erster Abschnitt **81,** der in Produktionsrichtung **W** vor der Abzweigung **30** liegt, und ein zweiter Abschnitt **82,** der in Produktionsrichtung **W** hinter der Abzweigung **30** liegt, wobei in diesen Abschnitten jeweils eine Grundbindung **70** vorliegen kann.

Der erste Abschnitt **81** und der zweite Abschnitt **82** werden nach dem Wirken des Gefäßimplantats **1** abgetrennt. Die Grundbindung **70** der Abschnitte **81, 82** geht in einem an die Abzweigung **30** benachbarten und/oder angrenzenden Bereich in Trennfäden **72** über. Dies hilft, ein Aufribbeln der Gewirkstruktur zu vermeiden. Zudem können die Abschnitte **81, 82** so leichter abgetrennt werden.

Die Abzweigung **30** selbst wird z.B. durch einen weiteren Abschnitt mit einer Grundbindung **70** bereitgestellt, wobei auch in diesem Bereich wieder eine vordere und eine hintere Textilfläche vorgesehen ist. Die vordere und die hintere Textilfläche können hierbei jeweils als Fortsetzung der vorderen Textilfläche **83** und/oder der hinteren Textilfläche **84,** die im Bereich des Hauptmantelkörpers **2** vorgesehen sind, ausgebildet sein. Die Grundbindung **70** entlang der Abzweigung **30** kann somit derjenigen der vorderen Textilfläche **83** und/oder derjenigen der hinteren Textilfläche **84** des Hauptmantelkörpers **2** entsprechen. Die jeweilige Unterlegung kann sich vom Hauptmantelkörper **2** in die Abzweigung **30** hinein erstrecken.

Wie ferner schematisch in **Figur 9** dargestellt ist, kann entlang der Abzweigung **30** benachbart und/oder angrenzend zu den Trennfäden **72** jeweils eine lokale Bindungsänderung **73** vorgesehen sein. Durch die lokale Bindungsänderung **73** werden die vordere und die hintere Textilfläche **83, 84** verbunden. Hierdurch entsteht auf einfache Weise im Bereich der Abzweigung **30** eine tubuläre Struktur.

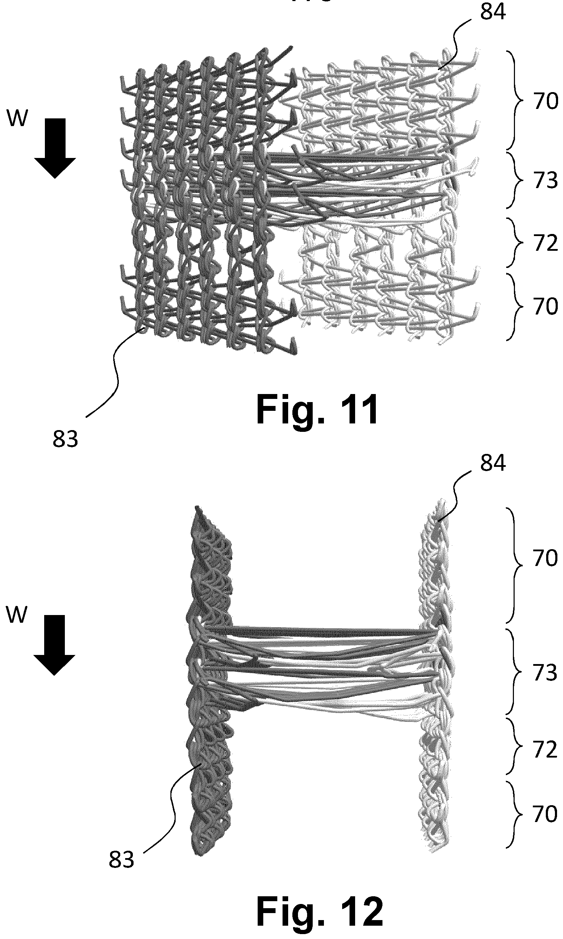

**Figuren 10 bis 12** zeigen schematisch das Detail A von **Figur 9** und damit ein Gewirk mit der besprochenen Bindungsabfolge entlang der vorderen Textilfläche **83** und der hinteren Textilfläche **84.** In Produktionsrichtung **W** gesehen folgt hierbei auf einen ersten Bereich mit Grundbindung **70** (entlang der Abzweigung **30**) eine lokale Bindungsänderung **73,** durch welche die vordere Textilfläche **83** und die hintere Textilfläche **84** zur Herstellung eines Tubus verbunden wird. Auf diese Bindungsänderung **73** folgen Trennfäden **72,** die einem Aufribbeln der Gewirkstruktur vorbeugen und nach Abschluss des Wirkprozesses die Abtrennung des überschüssigen Materials von der Abzweigung **30** (und damit vom Gefäßimplantat **1**) erleichtern. Anschließend können die vordere und die hintere Textilfläche **83, 84** entlang des Abschnitts **82,** der nach Abschluss des Wirkprozesses abgetrennt wird, wieder (zumindest abschnittsweise) mit einer Grundbindung **70** ausgebildet werden.

Die Bindungsanordnung von **Figuren 10 bis 12** zeigt den Bereich des Details A von **Figur 9** und somit das in Produktionsrichtung **W** gelegene Ende (d.h. den in Produktionsrichtung **W** gelegenen zweiten Randbereich) der Abzweigung **30.** Für den Fachmann wird jedoch erkennbar sein, dass eine entsprechende Bindungsanordnung auch an dem in Produktionsrichtung **W** gelegenen Anfang (d.h. an dem in Produktionsrichtung **W** gelegenen ersten Randbereich) der Abzweigung **30** vorgesehen sein kann, insbesondere eine Bindungsänderung in umgekehrter Reihenfolge.

Die Verwendung von lokalen Bindungsänderungen **73** in Verbindung mit Trennfäden **72** ist besonders vorteilhaft, wenn die vordere und die hintere Textilfläche **83, 84** in einem anderen Winkel als parallel zur Produktionsrichtung **W** miteinander verbunden werden sollen.

In **Figuren 9 bis 12** ist beispielhaft eine Bindungsanordnung für eine integral gewirkte Abzweigung **30** dargestellt, bei der die Abzweigung **30** und/oder deren Seitmantelkörper vom Hauptmantelkörper **2** in einem Winkel von etwa 90° entspringt. Die lokale Bindungsänderung **73** und der Übergang zu den Trennfäden **72** kann hierbei, wie in den Figuren dargestellt ist, im Wesentlichen senkrecht zur Produktionsrichtung **W** (d.h. in **Figuren 9 bis 12** und/oder bei üblichen Wirkmaschinen horizontal) verlaufen.

Die gezeigte Bindungsänderung und/oder Bindungsabfolge ist hierauf jedoch nicht beschränkt. So kann eine entsprechende Bindungsänderung und/oder Bindungsabfolge auch dann verwendet werden, wenn eine Abzweigung in einem anderen Winkel vom Hauptmantelkörper **2** entspringt und/oder bei anderen Strukturelementen, wie etwa einer oder mehrerer Durchmesseränderungen oder einer oder mehrerer Bifurkationen (auch hier, unabhängig vom Winkel, in dem diese schräg zum Hauptkörper verlaufen).

Statt einem Verlauf der lokalen Bindungsänderung **73** und/oder einem Verlauf des Übergangs auf die Trennfäden **72** im Wesentlichen senkrecht zur Produktionsrichtung **W** (horizontal) und/oder im Wesentlichen senkrecht zur Haupterstreckungsrichtung **12,** verläuft die lokale Bindungsänderung **73** und/oder der Übergang auf die Trennfäden **72** in diesem Fall vorzugsweise in einem entsprechenden Winkel zu der Produktionsrichtung **W** und/oder der Haupterstreckungsrichtung **12** (d.h. diagonal). Die prinzipielle Abfolge von Grundbindung - lokale Bindungsänderung (zum Verbinden der Flächen) - Trennfäden - Grundbindung kann hierbei beibehalten werden, würde bei Betrachtung entlang einer Achse senkrecht zur Produktionsrichtung **W** jedoch sukzessive in der Produktionsrichtung **W** versetzt (d.h. in Produktionsrichtung **W** sukzessive zu einem früheren oder späteren Zeitpunkt ausgebildet).

Eine solche diagonale Ausbildung der Bindungsänderungen ist in **Figur 13** schematisch für eine Abzweigung **30** dargestellt. Wie hierbei für den unteren Randbereich der Abzweigung **30** beispielhaft aufgezeigt wird, findet die Bindungsänderung (Grundbindung **70** - lokale Bindungsänderung **73** zum Verbinden der Flächen - Trennfäden **72** - Grundbindung **70** in Produktionsrichtung **W** jeweils mit einer leichten zeitlichen und/oder räumlichen Verzögerung statt, sodass die Bindungsänderung schräg ausgebildet wird. Wie der Fachmann erkennen wird, kann am oberen Randbereich eine entsprechende Bindungsabfolge erfolgen (dort in umgekehrter Reihenfolge). Falls gewünscht, kann der obere und/oder der untere Randbereich des Strukturelements auf diese Weise auch mit einem abgerundeten Verlauf versehen werden.

**Figur 14** zeigt schematisch die Verwendung einer solchen Bindungsabfolge zur Ausbildung einer Durchmesseränderung **10** (wobei zur Vereinfachung der Darstellung nur eine Hälfte des Gefäßimplantats **1** gezeigt ist). Wie für den Fachmann erkennbar sein wird, kann die Durchmesseränderung **10** achsensymmetrisch zur Haupterstreckungsachse **2**ausgebildet sein, muss dies jedoch nicht.

Um der Durchmesseränderung **10** einen schrägen Verlauf zu geben, wurde auch in diesem Fall eine Bindungsabfolge gewählt, die sich schräg zur Haupterstreckungsrichtung **12** sukzessive ändert.

In **Figur 14** ist eine Durchmesserverringerung in Produktionsrichtung **W** gezeigt, weshalb die Bindungsänderung in der Produktionsrichtung **W** zunächst an einer zur Haupterstreckungsrichtung **12** radial äußeren Stelle beginnt und dann sukzessive nach innen verläuft. Wie der Fachmann erkennen wird, kann jedoch auch eine Durchmesservergrößerung entsprechend hergestellt werden, wobei die Bindungsänderung in der Produktionsrichtung **W** dann zunächst an einer zur Haupterstreckungsrichtung **12** radial inneren Stelle beginnt und dann sukzessive nach außen verläuft.

### Bezugszeichenliste

- 1: Endovaskuläres Gefäßimplantat
- 2: Tubulärer Hauptmantelkörper
- 10: Durchmesseränderung
- 11: Länge des Gefäßimplantats
- 12: Haupterstreckungsrichtung
- 20: Bifurkation
- 21: Bifurkationslänge
- 22: Bifurkationslänge
- 23: Bifurkationswinkel
- 30: Abzweigung
- 31: Länge der Abzweigung
- 32: Abzweigungswinkel
- 40: Fenestrierung
- 50: Scallop
- 51: offenes Ende
- 52: Aussparungshöhe
- 53: Aussparungsbreite
- 60: Köperbindung
- 70: Grundbindung
- 71: Randabbindung
- 72: Trennfäden
- 73: Lokale Bindungsänderung
- 80: Abschnitt mit Unterlegung
- 81: erster Abschnitt
- 82: zweiter Abschnitt
- 83: vordere Textilfläche
- 84: hintere Textilfläche
- 90: Iliakalgefäß-Grafts
- 91: Trennfäden für Iliakalgefäß-Grafts
- 100: Verfahren zur Herstellung eines endovaskulären Gefäßimplantats
- 110: Erhalten von Gefäßparametern
- 120: Erstellen eines computergestützten Modells
- 130: Auswählen eines oder mehrerer Strukturelemente
- 140: Bestimmen von Parametern
- 150: Integrieren der Strukturelemente in das computergestützte Modell
- 160: Herstellen des Gefäßimplantats
- 170: Thermoformen und/oder Thermofixieren
- 180: Aufbringen einer Stentstruktur
- W: Produktionsrichtung

## Patentansprüche

1. Verfahren (100) zur Herstellung eines Gefäßimplantats (1), das folgende Schritte aufweist:
• Erhalten von Gefäßparametern (110);
• Erstellen eines computergestützten Modells eines Gefäßimplantats (1), wobei das Gefäßimplantat (1) ein oder mehrere Module mit jeweils mindestens einem tubulären Mantelkörper (2) aufweist, basierend auf den erhaltenen Gefäßparametern (120);
• Auswählen eines oder mehrerer Strukturelemente (130) eines jeweiligen Moduls aus der Gruppe bestehend aus: einer oder mehreren Durchmesseränderungen (10) entlang mindestens eines tubulären Mantelkörpers (2), einer oder mehreren Bifurkationen (20), einer oder mehreren Abzweigungen (30), einer oder mehreren Aussparungen (40, 50), einer oder mehreren lokalen Verstärkungen (60), und einem oder mehreren Iliakalgefäß-Grafts;
• Bestimmen von Parametern zu dem einen oder den mehreren ausgewählten Strukturelementen (140);
• Integrieren der Strukturelemente gemäß den bestimmten Parametern in das computergestützte Modell (150);
• Herstellen des Gefäßimplantats (1) anhand des erstellten computergestützten Modells (160),
**dadurch gekennzeichnet, dass**
das Gefäßimplantat (1) textil und/oder textilbasiert ist, insbesondere ein Gewebe und/oder eine Maschenware ist, und
wobei das Gefäßimplantat (1) mittels Jacquard-Technik hergestellt ist.

2. Verfahren nach Anspruch 1, wobei das Herstellen des Gefäßimplantats (1) ein Wirken des Gefäßimplantats (1) anhand des erstellten computergestützten Modells (160) umfasst.

3. Verfahren nach Anspruch 1 oder 2, wobei das Herstellen des Gefäßimplantats (1) ein einstückiges Wirken des Gefäßimplantats umfassend mindestens ein Modul mit jeweils mindestens einem tubulären Mantelkörper und jeweiligen ein oder mehreren Strukturelementen aufweist.

4. Verfahren nach einem der vorherigen Ansprüche, wobei das Gefäßimplantat (1) eine oder mehrere Bifurkationen (20) und/oder eine oder mehrere Abzweigungen (30) aufweist, wobei das Verfahren ferner die Schritte umfasst:
• vorzugsweise Bestimmen von Geometrie und Bindung am Übergang vom Hauptmantelkörper (2) zu der mindestens einen Bifurkation (20) und/oder mindestens einen Abzweigung (30);
• Bestimmen von Positionen von Trennfäden;
• Einbringen von Trennfäden bei der Herstellung des Gefäßimplantats (1); und
• Trennen der Trennfäden des hergestellten Gefäßimplantats (1).

5. Verfahren nach einem der vorherigen Ansprüche, wobei das Gefäßimplantat (1) eine oder mehrere Aussparungen (40, 50) aufweist, wobei das Verfahren ferner die Schritte umfasst:
• Bestimmen von Positionen von mindestens einer lokalen Verstärkung (60) an den Rändern der mindestens einen Aussparung (40, 50); und
• wenn mindestens eine Aussparung (40, 50) eine Fenestrierung (40) ist: Einbringen von mindestens einer lokalen Verstärkung (60) bei der Herstellung des Gefäßimplantats (1);
• wenn mindestens eine Aussparung (40, 50) ein Scallop (50) ist: Bestimmen von Positionen von Trennfäden; Einbringen von mindestens einer lokalen Verstärkung (60) und Einbringen von Trennfäden bei der Herstellung des Gefäßimplantats (1); Trennen der Trennfäden des hergestellten Gefäßimplantats (1).

6. Verfahren nach einem der vorherigen Ansprüche, wobei das Gefäßimplantat (1) eine oder mehrere Durchmesseränderungen (10) entlang mindestens eines tubulären Mantelkörpers (2) aufweist, wobei das Verfahren ferner die Schritte umfasst:
• Bestimmen von Parametern zur Fadenspannung, Fadenanzahl, Maschenreihendichte, und/oder Maschengröße.

7. Verfahren nach einem der vorherigen Ansprüche, wobei das Gefäßimplantat (1) eine oder mehrere Durchmesseränderungen (10) entlang mindestens eines tubulären Mantelkörpers (2) aufweist, wobei das Verfahren ferner die Schritte umfasst:
• Bestimmen von Positionen von Trennfäden; Einbringen von Trennfäden bei der Herstellung des Gefäßimplantats (1); und Trennen der Trennfäden des hergestellten Gefäßimplantats (1).

8. Verfahren nach einem der vorherigen Ansprüche, das ferner die Schritte aufweist:
• Erhalten pre-operativer medizinischer Bilddaten eines Gefäßes;
• Visualisieren der erhaltenen Bilddaten;
• Identifizieren eines Gefäßabschnittes, für den ein Gefäßimplantat (1) hergestellt werden soll; und
• Messen von Gefäßparametern.

9. Verfahren nach einem der vorherigen Ansprüche, wobei das computergestützte Modell ein 3D-Modell und/oder eine maschinenlesbare Datei umfasst, wobei der Schritt des Herstellens des Gefäßimplantats (1) vorzugsweise auf Basis der maschinenlesbaren Datei erfolgt.

10. Verfahren nach einem der vorherigen Ansprüche, das ferner das Aufbringen einer Stentstruktur auf das hergestellte Gefäßimplantat (1) und/oder einer Klappe an dem hergestellten Gefäßimplantat (1) umfasst, vorzugsweise wobei die Stentstruktur auf das Gefäßimplantat (1) gedruckt wird.

11. Verfahren nach einem der vorherigen Ansprüche, das ferner einen Schritt der Thermoformung und/oder Thermofixierung des Gefäßimplantats (1) zur Herstellung der einen oder der mehreren Durchmesseränderungen (10) aufweist.

12. Verfahren nach einem der vorherigen Ansprüche, das ferner einen Schritt der Thermoformung und/oder Thermofixierung des Gefäßimplantats (1) zur Aufweitung der einen oder mehreren Aussparungen (40, 50) aufweist.

13. Verfahren nach einem der vorherigen Ansprüche, das ferner einen Schritt der Thermoformung und/oder Thermofixierung des Gefäßimplantats (1) zur Veränderung und/oder Fixierung des Winkels einer oder mehrerer Bifurkationen (20) und/oder Abzweigungen (30) relativ zu einer Haupterstreckungsrichtung (2) des Gefäßimplantats (1).

14. Verfahren nach einem der vorherigen Ansprüche, wobei das Gefäßimplantat (1) ein endovaskuläres Gefäßimplantat (1) ist.

15. Gefäßimplantat (1), insbesondere endovaskuläres Gefäßimplantat (1), hergestellt mit dem Verfahren (100) gemäß einem der vorherigen Ansprüche.

## Claims

1. A method (100) for producing a vascular implant (1), comprising the following steps:
• obtaining vessel parameters (110);
• creating a computer-aided model of a vascular implant (1) based on the obtained vessel parameters (120), wherein the vascular implant (1) comprises one or more modules, each comprising at least one tubular casing body (2);
• selecting one or more structural elements (130) of a respective module from the group consisting of: one or more diameter changes (10) along at least one tubular casing body (2), one or more bifurcations (20), one or more branches (30), one or more recesses (40, 50), one or more local reinforcements (60) and one or more iliac vessel grafts;
• determining parameters relating to the one or more selected structural elements (140);
• integrating the structural elements into the computer-aided model according to the determined parameters (150);
• producing the vascular implant (1) on the basis of the created computer-aided model (160),
**characterized in that**
the vascular implant (1) is textile and/or textile-based, in particular a woven fabric and/or a knitted fabric, and
wherein the vascular implant (1) is produced using the jacquard technique.

2. The method according to claim 1, wherein producing the vascular implant (1) comprises knitting the vascular implant (1) on the basis of the created computer-aided model (160).

3. The method according to claim 1 or 2, wherein producing the vascular implant (1) comprises integrally knitting the vascular implant comprising at least one module each comprising at least one tubular casing body and respective one or more structural elements.

4. The method according to any one of the preceding claims, wherein the vascular implant (1) comprises one or more bifurcations (20) and/or one or more branches (30), wherein the method further comprises the steps of:
• preferably determining the geometry and weave at the transition from the main casing body (2) to the at least one bifurcation (20) and/or at least one branch (30);
• determining positions of separating threads;
• introducing separating threads during the production of the vascular implant (1); and
• separating the separating threads of the produced vascular implant (1).

5. The method according to any one of the preceding claims, wherein the vascular implant (1) comprises one or more recesses (40, 50), wherein the method further comprises the steps of:
• determining positions of at least one local reinforcement (60) at the edges of the at least one recess (40, 50); and
• when at least one recess (40, 50) is a fenestration (40): incorporating at least one local reinforcement (60) during the production of the vascular implant (1);
• when at least one recess (40, 50) is a scallop (50): determining positions of separating threads; incorporating at least one local reinforcement (60) and incorporating separating threads during the production of the vascular implant (1); separating the separating threads of the produced vascular implant (1).

6. The method according to any one of the preceding claims, wherein the vascular implant (1) comprises one or more diameter changes (10) along at least one tubular casing body (2), wherein the method further comprises the steps of:
• determining parameters relating to thread tension, thread count, course density and/or stitch size.

7. The method according to any one of the preceding claims, wherein the vascular implant (1) comprises one or more diameter changes (10) along at least one tubular casing body (2), wherein the method further comprises the steps of:
• determining positions of separating threads; incorporating separating threads during the production of the vascular implant (1); and separating the separating threads of the produced vascular implant (1).

8. The method according to any one of the preceding claims, further comprising the steps of:
• obtaining pre-operative medical image data of a vessel;
• visualizing the obtained image data;
• identifying a vessel section for which a vascular implant (1) is to be produced; and
• measuring vessel parameters.

9. The method according to any one of the preceding claims, wherein the computer-aided model comprises a 3D model and/or a machine-readable file, wherein the step of producing the vascular implant (1) is preferably performed on the basis of the machine-readable file.

10. The method according to any one of the preceding claims, further comprising the application of a stent structure onto the produced vascular implant (1) and/or of a valve at the produced vascular implant (1), preferably wherein the stent structure is printed onto the vascular implant (1).

11. The method according to any one of the preceding claims, further comprising a step of thermoforming and/or thermosetting the vascular implant (1) in order to produce the one or more diameter changes (10).

12. The method according to any one of the preceding claims, further comprising a step of thermoforming and/or thermosetting the vascular implant (1) in order to expand the one or more recesses (40, 50).

13. The method according to any one of the preceding claims, further comprising a step of thermoforming and/or thermosetting the vascular implant (1) in order to modify and/or fix the angle of one or more bifurcations (20) and/or branches (30) relative to a main direction of extension (2) of the vascular implant (1).

14. The method according to any one of the preceding claims, wherein the vascular implant (1) is an endovascular implant (1).

15. A vascular implant (1), in particular an endovascular implant (1), produced by means of the method (100) according to any one of the preceding claims.

## Revendications

1. Procédé (100) de fabrication d'un implant vasculaire (1), qui présente des étapes suivantes :
• de réception de paramètres vasculaires (110) ;
• de création d'un modèle assisté par ordinateur d'un implant vasculaire (1), dans lequel l'implant vasculaire (1) présente un ou plusieurs modules avec respectivement au moins un corps d'enveloppe tubulaire (2), sur la base des paramètres vasculaires (120) reçus ;
• de sélection d'un ou de plusieurs éléments de structure (130) d'un module respectif parmi le groupe constitué de : une ou plusieurs modifications de diamètre (10) le long d'au moins un corps d'enveloppe tubulaire (2), une ou plusieurs bifurcations (20), un ou plusieurs ramifications (30), un ou plusieurs évidements (40, 50), un ou plusieurs renforcements locaux (60), et une ou plusieurs greffes d'artère iliaque ;
• de définition de paramètres concernant l'un ou les plusieurs éléments de structure (140) sélectionnés ;
• d'intégration des éléments de structure selon les paramètres définis dans le modèle assisté par ordinateur (150) ;
• de fabrication de l'implant vasculaire (1) à l'aide du modèle assisté par ordinateur (160) créé,
**caractérisé en ce que**
l'implant vasculaire (1) est en textile et/ou à base de textile, en particulier est un tissu et/ou un article à mailles, et
dans lequel l'implant vasculaire (1) est fabriqué au moyen d'une technique Jacquard.

2. Procédé selon la revendication 1, dans lequel la fabrication de l'implant vasculaire (1) comprend le tricotage de l'implant vasculaire (1) à l'aide du modèle assisté par ordinateur (160) créé.

3. Procédé selon la revendication 1 ou 2, dans lequel la fabrication de l'implant vasculaire (1) comprenant le tricotage en une seule pièce de l'implant vasculaire présente au moins un module avec respectivement au moins un corps d'enveloppe tubulaire et respectivement un ou plusieurs éléments de structure.

4. Procédé selon l'une quelconque des revendications précédentes, dans lequel l'implant vasculaire (1) présente une ou plusieurs bifurcations (20) et/ou une ou plusieurs ramifications (30), dans lequel le procédé comprend en outre les étapes :
• de définition de préférence de la géométrie et du tissage au niveau de la transition du corps d'enveloppe principal (2) à l'au moins une bifurcation (20) et/ou l'au moins une ramification (30) ;
• de définition de positions de fils de séparation ;
• d'introduction de fils de séparation lors de la fabrication de l'implant vasculaire (1) ; et
• de séparation des fils de séparation de l'implant vasculaire (1) fabriqué.

5. Procédé selon l'une quelconque des revendications précédentes, dans lequel l'implant vasculaire (1) présente un ou plusieurs évidements (40, 50), dans lequel le procédé comprend en outre les étapes :
• de définition de positions d'au moins un renforcement local (60) sur les bords de l'au moins un évidement (40, 50) ; et
• lorsqu'au moins un évidement (40, 50) est une fenestration (40) : d'introduction d'au moins un renforcement local (60) lors de la fabrication de l'implant vasculaire (1) ;
• lorsqu'au moins un évidement (40, 50) est un feston (50) : de définition de positions de fils de séparation ; d'introduction d'au moins un renforcement local (60) et d'introduction de fils de séparation lors de la fabrication de l'implant vasculaire (1) ; de séparation des fils de séparation de l'implant vasculaire (1) fabriqué.

6. Procédé selon l'une quelconque des revendications précédentes, dans lequel l'implant vasculaire (1) présente une ou plusieurs modifications de diamètre (10) le long d'au moins un corps d'enveloppe tubulaire (2), dans lequel le procédé comprend en outre les étapes :
• de définition de paramètres concernant la tension de fil, le nombre de fils, la densité de rangées de mailles et/ou la taille de mailles.

7. Procédé selon l'une quelconque des revendications précédentes, dans lequel l'implant vasculaire (1) présente une ou plusieurs modifications de diamètre (10) le long d'au moins un corps d'enveloppe tubulaire (2), dans lequel le procédé comprend en outre les étapes :
• de définition de positions de fils de séparation ; d'introduction de fils de séparation lors de la fabrication de l'implant vasculaire (1) ; et de séparation des fils de séparation de l'implant vasculaire (1) fabriqué.

8. Procédé selon l'une quelconque des revendications précédentes, qui présente en outre les étapes :
• de réception de données d'image médicales préopératoires d'un vaisseau ;
• de visualisation des données d'image reçues ;
• d'identification d'un tronçon de vaisseau, pour lequel un implant vasculaire (1) doit être fabriqué ; et
• de mesure de paramètres vasculaires.

9. Procédé selon l'une quelconque des revendications précédentes, dans lequel le modèle assisté par ordinateur comprend un modèle 3D et/ou un fichier lisible par une machine, dans lequel l'étape de la fabrication de l'implant vasculaire (1) est effectuée de préférence sur la base du fichier lisible par une machine.

10. Procédé selon l'une quelconque des revendications précédentes, qui comprend en outre l'application d'une structure d'endoprothèse sur l'implant vasculaire (1) fabriqué et/ou un clapet sur l'implant vasculaire (1) fabriqué, de préférence dans lequel la structure d'endoprothèse est imprimée sur l'implant vasculaire (1).

11. Procédé selon l'une quelconque des revendications précédentes, qui présente en outre une étape de thermoformage et/ou de thermofixation de l'implant vasculaire (1) pour fabriquer l'une ou les plusieurs modifications de diamètre (10).

12. Procédé selon l'une quelconque des revendications précédentes, qui présente en outre une étape de thermoformage et/ou de thermofixation de l'implant vasculaire (1) pour élargir l'un ou les plusieurs évidements (40, 50).

13. Procédé selon l'une quelconque des revendications précédentes, qui présente en outre une étape de thermoformage et/ou de thermofixation de l'implant vasculaire (1) pour modifier et/ou fixer l'angle d'une ou de plusieurs bifurcations (20) et/ou d'une ou de plusieurs ramifications (30) par rapport à une direction d'extension principale (2) de l'implant vasculaire (1).

14. Procédé selon l'une quelconque des revendications précédentes, dans lequel l'implant vasculaire (1) est un implant endovasculaire (1).

15. Implant vasculaire (1), en particulier implant endovasculaire (1), fabriqué avec le procédé (100) selon l'une quelconque des revendications précédentes.
